# EUROPEAN PATENT SPECIFICATION

(11) **EP 4 205 708 B1**
(45) Date of publication and mention of the grant of the patent: **22.01.2025**
(21) Application number: 22205206.0
(22) Date of filing: 19.08.2022
(51) Int. Cl.: A61B 18/20, A61B 18/00, A61N 1/04, A61N 1/32, A61N 5/06, A61F 7/00

(54) **SKIN CARE ASSEMBLY**
HAUTPFLEGEANORDNUNG
ENSEMBLE DE SOIN DE LA PEAU

(30) Priority: 11.03.2022 CN 202210238926; 24.09.2021 CN 202111124323; 11.03.2022 CN 202210239671; 11.03.2022 CN 202210239682; 11.03.2022 CN 202210238927
(43) Date of publication of application: 05.07.2023
(62) Divisional of application: 22789823.6
(73) Proprietor: Shenzhen Ulike Smart Electronics Co., Ltd., Shenzhen, Guangdong 518000 (CN)
(72) Inventor: YU, Fei, Shenzhen, Guangdong (CN); LIN, Xiaoming, Shenzhen, Guangdong (CN); PAN, Yuping, Shenzhen, Guangdong (CN)
(74) Representative: Page White Farrer

(56) References cited:
- CN-U- 212 941 026
- CN-U- 213 822 400
- KR-B1- 102 123 667
- US-A1- 2016 324 719
- US-A1- 2022 125 672
- US-B2- 10 835 447

## Description

### TECHNICAL FIELD

The present disclosure relates to the field of beauty device technologies, and in particular to a skin care assembly.

### BACKGROUND

With scientific and technological progress and development of the times, people pay more attention to skin care. In addition, when paying attention to the skin care effect, people also pay more attention to the experience of caring skin. A method of the caring skin is a skin care assembly which utilizes radio frequency or microcurrent.

In the related art, during a process of using the skin care assembly, the skin can only be heated continuously. However, when the temperature of the epidermis rises to a certain level, a tingling feeling will be caused, which brings discomfort to the user. At the same time, during a process of stimulating the skin, the epidermis and dermis are always at high temperature, and the skin cannot be expanded and contracted, such that the caring effect or beauty effect are not improved significantly.

CN213822400U discloses a cold and hot scraping therapy beauty instrument. A positive voltage or a negative voltage is applied to the refrigeration sheet through the mainboard, and the contact surface of the refrigeration sheet and the metal beauty piece is controlled to absorb heat or release heat, so that the metal beauty piece is refrigerated or heated; the cold and hot scraping therapy beauty instrument has the hot compress function and the cold compress function at the same time.

CN212941026U discloses a multifunctional facial beautification instrument. The device includes a handle, the beautifying machine head is arranged on the handle; the beautifying electrode assembly is used for conducting electricity, heating, refrigerating and vibrating; the semiconductor cold and hot assembly is used as a cold source or a heat source and transmits heat or cold energy to the cosmetic electrode assembly; and a vibration assembly serving as a high-frequency vibration source to vibrate the cosmetic electrode assembly. The physical therapy light source assembly is used for generating physical therapy light, and the physical therapy light is emitted from the beauty electrode assembly and irradiates the skin of a user; and the circuit board is arranged in the handle and used for controlling the cosmetic electrode assembly, the physiotherapy light source assembly, the vibration assembly and the semiconductor cold and hot assembly to work.

US20160324719A1 discloses a portable compress device includes a main body, a power source, an electric means for generating a temperature gradient on a plurality of surfaces, a controller means coupled with the electric means and the power source, a thermal sensor device configured to be in proximity to a user's skin at a treatment area, and a plurality of pad attachment and detachment means for attaching and detaching a pad assembly to and from the plurality of surfaces, wherein the pad assembly is for moistened or dry application on the treatment area.

US2022125672A1 discloses cooling attachment module for use with a vibration therapy device that includes a housing and/or heat sink member that includes inner and outer walls and defines a central opening axially therethrough. A cooling recess is defined in an upper surface and a connection recess is defined in a lower surface of the heat sink member. A cover member is secured over the cooling recess. A controllable temperature element is positioned on an upper surface of the heat sink member. A spreader member is positioned on an upper surface of the controllable temperature element. An upper surface of the spreader member is positioned above an upper surface of the cover member to contact a user's body part. The controllable temperature element is configured to transfer thermal energy to a lower surface of the spreader member. A base portion that includes an electrical connector is secured under the electrical recess.

US10835447B2 discloses a handheld massage tool that provides a cooling effect, intended to treat and improve the appearance of the skin, especially of the face.

KR102123667B1 relates to a skin care device having improved thermal conductivity and heat dissipation properties. The skin care device having improved thermal conductivity and heat dissipation properties includes: a main body including a base body, a front surface cover, and a head; a thermoelectric element unit; a heat dissipation unit including a heatsink and a heat dissipation fan; an ultrasonic wave generation unit; and a vibrating unit.

### SUMMARY

The invention is set out in the appended set of claims.

In the embodiments of the present disclosure, the skin care assembly may achieve a good use comfort and excellent beauty effect. An electrode head and the cold compress are relatively fixed, such that it is possible to reduce redundant structures and make the design more concise. At the same time, there is no interference of redundant structures, and the touch of the skin is also more comfortable. The electrode head provides beauty functions such as radio frequency and micro-current. A suitable temperature environment is provided by the cooperation of cold compress member and the refrigeration member, and the heat dissipation member, which further improves the beauty effect.

### BRIEF DESCRIPTION OF THE DRAWINGS

In order to more clearly describe the technical solutions in the embodiments of the present disclosure, the drawings that need to be used in the description of the embodiments will be briefly described in the following. Apparently, the drawings in the following description are only some embodiments of the present disclosure. For those skilled in the art, other drawings can be obtained based on these drawings without creative work.
FIG. 1 is an entire structural schematic view of a skin care assembly according to an embodiment of the present disclosure.
FIG. 2 is a cross-sectional view of the skin care assembly along A-A direction shown in FIG. 1.
FIG. 3 is a partial enlarged schematic view of the skin care assembly as shown in FIG. 2 at a position A.
FIG. 4 is a structural schematic view of an electrode and a cold compress member of the skin care assembly according to an embodiment of the present disclosure.
FIG. 5 is a structural schematic view of the electrode of the skin care assembly according to an embodiment of the present disclosure.
FIG. 6 is an exploded schematic view in a direction of the skin care assembly according to an embodiment of the present disclosure.
FIG. 7 is an exploded schematic view in another direction of the skin care assembly as shown in FIG. 6.
FIG. 8 is a structural schematic view of the skin care assembly according to an embodiment of the present disclosure.
FIG. 9 is a cross-sectional view of the skin care assembly along A-A direction shown in FIG. 8.
FIG. 10 is an exploded schematic view of an operation head according to an embodiment of the present disclosure.
FIG. 11 is a structural schematic view of a heat dissipation member and a heat pipe according to an embodiment of the present disclosure.
FIG. 12 is a structural schematic view in a direction of the heat dissipation member as shown in FIG. 11.
FIG. 13 is an exploded schematic view in another direction of a beauty device according to an embodiment of the present disclosure.
FIG. 14 is a perspective exploded schematic view of the skin care assembly according to an embodiment of the present disclosure.
FIG. 15 is a perspective exploded schematic view of the skin care assembly according to another embodiment of the present disclosure.
FIG. 16 is a front structural schematic view of the skin care assembly according to another embodiment of the present disclosure.
FIG. 17 is a cross-sectional structural schematic view of the skin care assembly according to another embodiment of the present disclosure.
FIG. 18 is a perspective exploded structural schematic view of the skin care assembly according to some embodiments of the present disclosure viewed from a view angle.
FIG. 19 is a perspective exploded structural schematic view of the skin care assembly according to some embodiments of the present disclosure viewed from another view angle.
FIG. 20 is a partial exploded structural schematic view of the skin care assembly according to an embodiment of the present disclosure.
FIG. 21 is a partial exploded structural schematic view of a connection member of the skin care assembly according to another embodiment of the present disclosure.
FIG. 22 is a partial exploded structural schematic view of the skin care assembly according to another embodiment of the present disclosure.
FIG. 23 is a cross-sectional structural schematic view of the skin care assembly according to an embodiment of the present disclosure.
FIG. 24 is a side schematic view of a cold compress member as shown in FIG. 23.
FIG. 25 is a partial enlarged schematic view of the skin care assembly as shown in FIG. 23 at a position A.
FIG. 26 is a perspective structural schematic view of the electrode of the skin care assembly as shown in FIG. 23.
FIG. 27 is an installation schematic view of a first cover, a second cover, and a heat dissipation member as shown in FIG. 23.
FIG. 28 is a cross-sectional structural schematic view of the skin care assembly according to another embodiment of the present disclosure.
FIG. 29 is a side schematic view of the cold compress member as shown in FIG. 28.
FIG. 30 is a partial enlarged schematic view of the skin care assembly as shown in FIG. 28 at a position B.
FIG. 31 is an exploded structural schematic view of the skin care assembly according to another embodiment of the present disclosure.
FIG. 32 is a cross-sectional structural schematic view of a contact-type skin care device according to an embodiment of the present disclosure.
FIG. 33 is an exploded structural schematic view of the skin care assembly according to another embodiment of the present disclosure.
FIG. 34 is a structural schematic view of a light guide plate of the skin care assembly according to an embodiment of the present disclosure, showing a side of the light guide plate close to a phototherapeutic light board.
FIG. 35 is a cross-sectional schematic view of the skin care assembly according to another embodiment of the present disclosure.
FIG. 36 is an exploded schematic view of the first cover, the light guide plate, the phototherapeutic light board, and a first bolt of the skin care assembly according to an embodiment of the present disclosure.
FIG. 37 is a cross-sectional schematic view of the first cover, the light guide plate, the phototherapeutic light board, and the first bolt of the skin care assembly in an assembled state according to an embodiment of the present disclosure.

### DETAILED DESCRIPTION

In order to make the above-mentioned objects, features and advantages of the present disclosure more obvious and easy to understand, embodiments of the present disclosure will be described in detail below with reference to the accompanying drawings. It should be understood that embodiments described herein are only used to explain the present disclosure, but not to limit the present disclosure. In addition, it should be noted that, for the convenience of description, the drawings only show some but not all the structures related to the present disclosure.

The terms "first", "second", and so on in the present disclosure are intended for distinguishing different objects, rather than describing a specific order. In addition, the terms "include" and "have" and any variations thereof are intended to cover non-exclusive inclusion. For example, a process, method, system, product, or apparatus including a series of steps or units is not limited to the listed steps or units, but optionally also includes steps or units not listed, or optionally also includes other steps or units inherent to the process, method, product, or apparatus.

References herein to "embodiments" mean that particular features, structures, or characteristics described in connection with an embodiment may be included in at least one embodiment of the present disclosure. The presence of the phrase at various points in the specification does not necessarily mean a same embodiment, nor is it a separate or alternative embodiment that is mutually exclusive with other embodiments. It is understood, both explicitly and implicitly, by those skilled in the art that the embodiments described herein may be combined with other embodiments.

A technical solution adopted by the present disclosure is to provide a skin care assembly. The skin care assembly includes: a cold compress member including a contact surface and a conduction surface, wherein the contact surface is configured to contact a skin; a refrigeration member and a heat dissipation member, wherein a cooling side of the refrigeration member is thermally coupled with the conduction surface of the cold compress member to cool the cold compress member, and wherein the heat dissipation member is thermally coupled with a heat generating side of the refrigeration member to dissipate heat generated by the refrigeration member; a first cover configured to relatively fix the cold compress member to the heat dissipation member; and one or more electrodes relatively fixed with the first cover, wherein each of the one or more electrodes includes a main body portion and a fixing portion connected to the main body portion, wherein the main body portion is configured to contact the skin, and wherein each of the one or more electrodes is fixed with the first cover via the fixing portion.

In some embodiments, an installation port is defined on the fixing portion, and a diameter of a projection of the installation port on a plane which the cold compress member is located on is smaller than that of a projection of the main body portion on the plane which the cold compress is located on; and the main body portion is at least partially exposed from the installation port.

In some embodiments, the main body portion is a sphere and a conductor.

In some embodiments, the skin care assembly further includes an elastic member. The elastic member is arranged in the fixing portion and located between the main body portion and a bottom surface of the fixing portion.

In some embodiments, the elastic member abuts against the bottom surface of the fixing portion and the main body portion, and the main body portion is pressed against the installation port under an action of the elastic member.

In some embodiments, the main body portion, the elastic member, and the fixing portion are conductors.

In some embodiments, the skin care assembly further includes a vibrating generator. The electrode vibrates at a certain frequency under an action of the vibrating generator.

In some embodiments, the number of the electrodes is at least two, and the at least two electrodes are arranged on the first cover and spaced apart from each other. The first cover defines an opening, and a first stepped surface is disposed on the opening, a second stepped surface is disposed on a side of the cold compress member close to the opening, the first stepped surface abuts against the second stepped surface, and the second stepped surface is located between the first cover and the refrigeration member.

In some embodiments, the skin care assembly further includes a phototherapeutic light disposed opposite to the cold compress member. The phototherapeutic light is configured to emit light to an outside of the cold compress member.

In some embodiments, the skin care assembly further includes a phototherapeutic light board. The phototherapeutic light board includes one or more phototherapeutic lights, and the one or more phototherapeutic lights are relatively fixed with the first cover.

In some embodiments, the skin care assembly further includes a light guide plate. The light guide plate includes one or more light guide holes, the light emitted by the phototherapeutic light is reflected on the cold compress member via the one or more light guide holes, the light guide plate is disposed between the first cover and the phototherapeutic light, and the light guide plate is relatively fixed with the first cover.

In some embodiments, the number of the electrodes is at least two, and the at least two electrodes are arranged on a peripheral side of the cold compress member and spaced apart from each other. The first cover abuts against the cold compress member via the electrode, so as to relatively fix the cold compress member to the heat dissipation member. An electromagnetic pulse includes a radio frequency and/or a micro current.

In some embodiments, a third stepped surface is disposed on a side of the electrode towards the cold compress member, a fourth stepped surface is disposed on a side of the cold compress member, the fourth stepped surface is located between the third stepped surface and the heat dissipation member, and the third stepped surface abuts against the fourth stepped surface.

In some embodiments, the first cover defines an opening, and a fifth stepped surface is disposed on the opening, a sixth stepped surface is disposed on the side of the cold compress member, the sixth stepped surface is opposite to the fourth stepped surface, the sixth stepped surface is located between the fifth stepped surface and the electrode, and the fifth stepped surface abuts against the sixth stepped surface.

In some embodiments, the cold compress member is pressed by the electrode at the opening of the first cover, and the cold compress member abuts against the electrode and the first cover respectively in two opposite directions.

In some embodiments, the fixing portion includes at least one installation post, the first cover defines at least one installation hole, and the installation post is installed in the installation hole to fix the electrode on the first cover. A height of the electrode protruding from the first cover is in a range from 0mm to 20mm.

In some embodiments, the electrode is exposed from the contact surface in a first direction, and the conduction surface passes through and is exposed from the opening.

In some embodiments, the skin care assembly further includes a cold-guiding member. The cold-guiding member is disposed between the cold compress member and the refrigeration member, and configured to conduct heat between the cold compress member and the refrigeration member. The cold-guiding member abuts against the cold compress member.

In some embodiments, the skin care assembly further includes a second cover. At least part of the second cover is disposed between the first cover and the heat dissipation member. The second cover is fixed on the heat dissipation member, and the first cover is fixed on the second cover. The cold-guiding member and the refrigeration member are pressed against the heat dissipation member in the first direction via the cold compress member.

In some embodiments, the skin care assembly further includes: a first fastener disposed on the first cover and a second fastener disposed on the second cover. The second fastener is snap-connected to the first fastener to fix the first cover to the second cover.

In some embodiments, a restriction hole is defined on the second cover, the restriction hole is located on and in communication with the opening, the refrigeration member is partially disposed in the restriction hole, and a distance between an edge of the refrigeration member and an edge of the restriction hole is in a range from 0.5mm to 1mm. A recess is defined on an area of the heat dissipation member corresponding to the refrigeration member, the recess is arranged corresponding to the restriction hole, one part of the refrigeration member is embedded in the recess, and the other part of the refrigeration member is disposed in the restriction hole. A ratio of an area of the restriction hole to an area of the opening is in a range from 0.8 to 1.2.

In some embodiments, the heat dissipation member includes a thermal coupling surface. The thermal coupling surface is configured to be thermally coupled with a heat generating surface of an operation head to dissipate heat generated by the operation head. The operation head includes the cold compress member and the refrigeration member. The heat dissipation member extends from a head end of the skin care assembly to a trailing end of the skin care assembly.

In some embodiments, a gap is defined between an ending of the heat dissipation member disposed on a trailing end of the skin care assembly and an ending of the trailing end of the skin care assembly, and a size of the gap is less than one third of a length of a device body.

In some embodiments, an air outlet is defined on a trailing end of the skin care assembly, and a trailing end of the heat dissipation member is arranged corresponding to the air outlet.

In some embodiments, a thermal coupling surface is disposed on an end surface of a head of the heat dissipation member. A recess is defined on the end surface, and a bottom surface of the recess is a thermal coupling surface. The heat generating side of the refrigeration member is a heat generating surface, and the heat generating surface is thermally coupled with the heat dissipation member to dissipate heat generated by the refrigeration member. The refrigeration member is disposed in the recess, and the cooling side is higher than the recess.

In some embodiments, a ratio of a thickness of the refrigeration member to a depth of the recess is in a range from 2 to 10.

In some embodiments, the skin care assembly further includes: an installation plate installed on the opening of the recess and extending towards the trailing end of the skin care assembly; and a charging port disposed on an end of the installation plate and exposed from the skin care assembly.

In some embodiments, the skin care assembly further includes a heat pipe. A section of the heat pipe is bent to form a bending portion, the bending portion abuts against an end of the heat dissipation member close to the operation head. The heat pipe has a hollow and sealing structure and is filled with liquid, and the liquid is not full of the heat pipe.

In some embodiments, an avoidance groove is defined on the heat dissipation member, the heat pipe is arranged along an inner wall of the avoidance groove and welded to the inner wall of the avoidance groove, and an end of the heat pipe away from the bending portion extends to the end of the heat dissipation member.

In some embodiments, the heat dissipation member is integrally formed.

In some embodiments, the refrigeration member is disposed between the heat dissipation member and the cold compress member to form a sandwich structure of the heat dissipation member, the refrigeration member, and the cold compress member.

In some embodiments, an opening is defined on the first cover, a size of the opening in at least one radial direction is smaller than a size of the cold compress member in the radial direction, the opening of the first cover is arranged corresponding to the cold compress member, and an edge of the cold compress member is pressed to the heat dissipation member through an edge of the opening, such that the cold compress member and the refrigeration member are fixed on the heat dissipation member.

In some embodiments, the electrode is disposed on the edge of the opening of the first cover, the number of the electrodes is at least two, the at least two electrodes are arranged on an outer periphery of the cold compress member and spaced apart from each other.

In some embodiments, the skin care assembly further includes: a first fastener disposed on the first cover and a second fastener disposed on the heat dissipation member. The second fastener is snap-connected to the first fastener; such that the cold compress member and the refrigeration member are fixed on the heat dissipation member.

In some embodiments, the heat dissipation member includes a thermal coupling surface thermally coupled with the refrigeration member and two side surfaces connected to two sides of the thermal coupling surface. A part of the second fastener is disposed a side of the thermal coupling surface of the heat dissipation member and snap-connected to the first fastener, a remaining part of the second fastener extends towards the two side surfaces of the two sides of the thermal coupling surface, and a guidance portion is arranged on an outside of the second fastener. The first cover includes two lateral wings corresponding to the two side surfaces of the heat dissipation member, and the two lateral wings of the first cover is assembled with the heat dissipation member via the guidance portion.

In some embodiments, one of the two lateral wings of the first cover is arranged with one of a guidance groove and a guidance rib, and the guidance groove or the guidance rib extends towards the heat dissipation member along the inner side of the first cover. One of the two side surfaces of the heat dissipation member is arranged with the other one of the guidance groove and the guidance rib, and the guidance groove is assembled with the guidance rib in a sliding manner. The other one of the two lateral wings of the first cover and the other one of the two side surfaces of the heat dissipation member are smooth.

In some embodiments, the heat dissipation member includes a second cover and a heat dissipation body, the second cover is disposed between the heat dissipation body and the first cover, the second cover is fixed on the heat dissipation body, and the first cover is fixed on the second cover.

In some embodiments, the skin care assembly further includes a lead connected to the refrigeration member. A recess is defined on the second cover, and the lead is accommodated in the recess.

In some embodiments, the guidance portion and the second fastener are disposed on the second cover, a restriction hole is defined on the second cover, the restriction hole is located on the opening and is in communication with the opening, the refrigeration member is partially disposed in the restriction hole, and a distance between an edge of the refrigeration member and an edge of the restriction hole is in a range from 0.5mm to 1mm. The recess is disposed along an edge of the restriction hole, and a ratio of an area of the restriction hole to an area of the opening is in a range from 0.8 to 1.2.

In some embodiments, the cold compress member includes a cold compress sheet and a cold-guiding sheet. The cold-guiding sheet is disposed between the cold compress sheet and the refrigeration member, the contact surface is disposed a side of the cold compress sheet away from the refrigeration member, the conduction surface is disposed a side of the cold-guiding sheet away from the cold compress sheet, a size of the cold-guiding sheet in at least one radial direction is greater than a size of the opening in the radial direction, and an edge of the cold-guiding sheet is pressed to the heat dissipation member by an edge of the opening of the first cover.

In some embodiments, the cold compress sheet is disposed in the opening, the cold compress sheet has a first radial dimension and a second radial dimension different from the first radial dimension at different thickness positions, and the opening has a third radial dimension and a fourth radial dimension different from the third radial dimension at different depth positions. A part of the cold compress sheet having the first radial dimension is closer to the cold-guiding sheet than a part of the cold compress sheet having the second radial dimension, and a part of the opening having the third radial dimension is closer to the cold-guiding sheet than a part of the opening having the fourth radial dimension. The first radial dimension is smaller than the third radial dimension, the second radial dimension is smaller than the fourth radial dimension, and the second radial dimension is greater than the third radial dimension.

In some embodiments, the cold compress member is made of sapphire. An area of the contact surface of the cold compress member is in a range from 350 square millimeters to 450 square millimeters, a thickness of the contact surface is in a range from 2mm to 5mm, and a ratio of the contact area to the thickness is in a range from 88:1 to 225:1.

In some embodiments, a ratio of an area of the contact surface of the cold compress member to a total area of a vertical projection of all the electrodes on a reference plane which the contact surface of the cold compress member is located on is in a range from 3.5:1 to 5:1, and a total power during discharge of the electrodes is in a range from 5 watts to 20 watts.

In some embodiments, a surface layer of the skin or skin tissue close to the skin is cooled by the cold compress member, an action depth range of electricity, magnetism or electromagnetism on the skin is larger than a depth of the surface layer. At the same time, an area of a cooling surface is greater than an action area of electricity, magnetism or electromagnetism on the skin. A working power of the cold compress member meets the following condition: the surface temperature is reduced to a range from 15°C to 45°C.

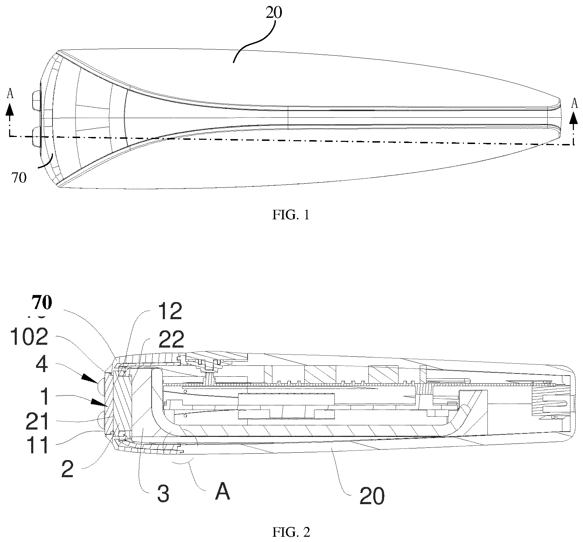

As shown in FIGS. 1-4, a skin care assembly includes a cold compress member 1, a refrigeration member 2, a heat dissipation member 3, and one or more electrodes 4. An operation port 102 is defined on a side of a support 70 away from a shell 20. The cold compress member 1 is assembled on the support 70, and the operation port 102 is covered by the cold compress member 1. Optionally, the cold compress member 1 includes a contact surface 11 and a conduction surface 12. The contact surface 11 faces the operation port 102, and the conduction surface 12 faces the refrigeration member 2. The contact surface 11 is configured to contact biological parts or skin. The contact surface 11 is exposed from the operation port 102, thereby better contacting the biological parts. The refrigeration member 2 has a cooling side 21, and the cooling side 21 of the refrigeration member 2 is thermally coupled with the conduction surface 12 of the cold compress member 1, thereby cooling the cold compress member 1. Optionally, the refrigeration member 2 has a heat generating side 22 thermally coupled with the heat dissipation member 3 to dissipate heat generated by the refrigeration member 2.

Optionally, each of the one or more electrodes 4 includes a fixing portion 41 and a main body portion 42. The main body portion 42 is installed on the fixing portion 41 in a rolling manner. The fixing portion 41 is relatively fixed with the cold compress member 1. The fixing portion 41 is assembled on a side of the cold compress member 1 away from the refrigeration member 2. The main body portion 42 is arranged in a rolling manner relative to the cold compress member 1. When the biological parts are beatified, the main body portion 42 is contacted with the skin in a rolling manner, such that when the one or more electrodes 4 moves on the biological parts, a frictional force may be reduced, thereby reducing the pulling between the each of the one or more electrodes 4 and the biological parts or scratching the skin, thus the convenience of using a beauty device is improved. At the same time, the main body portion 42 further provides a certain massaging function to relieve muscle fatigue, such that the skin care assembly has good performance.

Optionally, the skin care assembly may include a main body of the beauty device and an operation head. The main body of the beauty device may be the shell 20. The operation head may include the support 70, the one or more electrodes 4, the cold compress member 1, and so on. In a state of the support 70 connected with the shell 20, the heat dissipation member 3 is installed in the shell 20. The cold compress member 1 and the refrigeration member 2 are compressed via the support 70. The heat generating side 22 of the refrigeration member 2 abuts against the heat dissipation member 3. The conduction surface 12 of the cold compress member 1 abuts against the cooling side 21 of the refrigeration member 2. In this way, the operation head and the main body of the beauty device may be formed a whole, which may be used normally.

When the skin care assembly is in operation, the one or more electrodes 4 are discharged, for example, to generate radio frequency or micro-current. The current passes through and stimulates the biological parts, such that a desired beauty effect may be achieved. Since the biological parts is equivalent to an electrical resistance, a temperature of the biological parts may increase. The cold compress member 1, the refrigeration member 2, and the heat dissipation member 3 are thermally coupled with each other, such that the heat may be conducted from the biological parts to the heat dissipation member 3, thereby dissipating the heat from the beauty device. The cold compress member 1 is relatively fixed with the each of the one or more electrodes 4, the cold compress member 1 and the one or more electrodes 4 contact with the skin, and the cold compress member 1 is adjacent to the each of the one or more electrodes 4. When the biological parts located deeper is heated by the one or more electrodes 4, the biological parts located shallower is cooled by the cold compress member 1 in time, thereby improving touch for the biological parts.

A muscle contraction movement is caused when the current passes through the skin, thereby achieving the beauty effect. During a discharge process of the one or more electrodes 4, the skin temperature and the dermal temperature increase. The skin temperature is increased due to heat generated by skin cells and heat converted from electrical energy. The heat generated by the one or more electrodes 4 stimulating the epidermis is reduced via the cold compress member 1, thereby cooling the skin epidermis, and achieving a cold compress and cooling function. In this way, it is possible to reduce the situation that the skin is burned due to excessive temperature, thereby reducing the discomfort during use and improving the operating comfort of the operation head. The cold compress member 1 is cooled by the refrigeration member 2, such that the skin epidermis may be continuously cooled by the cold compress member 1. During a process of alternating hot and cold, it is also possible to expand contract the skin, such that the skin's breathing is enhanced, thereby protecting the skin from damage and better stimulating the tightening and regeneration of dermal collagen, tightening the skin, and improving beauty effect.

Optionally, the number of the electrodes 4 is at least two. In some embodiments of the present disclosure, the number of the electrodes 4 is four, but the number of the electrodes 4 is not limited herein. The four electrodes 4 are disposed on the cold compress member 1 in a matrix manner, and through the four electrodes 4, such that the biological parts may be fully beautified and the beauty effect may be improved via setting the four electrodes 4.

Optionally, the cold compress member 1 may be sapphire. The cold compress member 1 may be also a material with good thermal conductivity, such as a cold compress crystal, a cold compress glass, or the like. In some embodiments of the present disclosure, the sapphire is taken as example, but the material of the cold compress member 1 is not limited herein. The sapphire has a good heat transmission performance. The flake sapphire may conduct the heat generated by the one or more electrodes 4 on the biological parts fast to the refrigeration member 2, such that the temperature of the biological parts may be decreased rapidly to protect the skin health, thereby effectively preventing the skin from the damage.

An installation relationship is between the each of the one or more electrodes 4 and the cold compress member 1. Optionally, the each of the one or more electrodes 4 installed on the cold compress member 1 may be distributed in the center of the cold compress member 1, or may be distributed along a circumference. The one or more electrodes 4 and the cold compress member 1 contact with the biological parts. Since the one or more electrodes 4 are installed an entire piece of the sapphire, it is not necessary to install additional structures, such that there are no excess structures, such as a groove, a recess, a protrusion, an edge, or the like, to scratch the skin. Therefore, the one or more electrodes 4 are installed on the entire piece of the sapphire, which has comfort and heat transmission performances, thereby improving the comport of the contacted biological parts. When the beauty device is in operation, an end surface of the each of the one or more electrodes 4 and the contact surface 11 of the cold compress member 1 may stably contact the skin at the same time. Therefore, the each of the one or more electrodes 4 is directly installed on the cold compress member 1, which has comfort and conduction performances.

Optionally, an area of the contact surface 11 of the cold compress member 1 is in a range from 350 square millimeters to 450 square millimeters, a thickness of the cold compress member 1 is in a range from 2mm to 5mm, and a ratio of the contact area to the thickness is in a range from 88:1 to 225:1. For example, the area of the contact surface 11 of the cold compress member 1 may be 352 square millimeters, 440 square millimeters, or 450 square millimeters. For example, the thickness of the cold compress member 1 may be 2mm, 4mm, or 5mm. In this way, the area of the contact surface 11 of the cold compress member 1 may be optimized, and the thickness of the cold compress member 1 may be optimized, thereby further cooling the cold compress member 1.

For example, a total power during discharge of the electrodes 4 is in a range from 3 watts to 10 watts. When the beauty device performs the radio frequency function, the dermal temperature of the biological parts is in a range from 60°C to 65°C, and the skin temperature is in a range from 40°C to 43°C. A specific heat capacity in a range from 40°C to 43°C of the sapphire is 0.8 joules per gram Celsius. The end surface of the each of the one or more electrodes 4 is located on the side of the contact surface 11 of the cold compress member 1, and is located within a closed circle defined by an outer boundary of the contact surface 11, which means that the heat generated by the biological parts due to the each of one or more electrodes 4 with a relatively small area of the end surface may be conducted radially to the surrounding area through the contact surface 11 with a relatively large area. In this way, the contact surface 11 may be designed to be small enough to save costs while ensuring the desired cooling effect without causing a large area of cold feeling.

Before the one or more electrodes 4 contacts the skin, the temperature of the sapphire is lower than 20°C after the beauty device works for 30 seconds. The temperature of the sapphire is lower than 15°C after the beauty device works for 1 minute. Further, the temperature of the sapphire is lower than 10°C after the beauty device works for 1 minute and 30 seconds. It may be seen that the sapphire may play a good role in cooling the skin during continuous work. When contacting the biological parts, the beauty device may keep the epidermis in a comfortable temperature range continuously.

The cold compress member 1 with a suitable size may better transmit heat. At the same time, the cold compress member 1 may also ensure that the temperature of the biological parts may reach a balance when the beauty device is in operation, such that the temperature is not too high or too low. If the temperature is too high, the skin cells may be damage, resulting in a feeling of heat and pain. If the temperature is too low, the beauty effect cannot be achieved, resulting in a feeling of freezing.

Optionally, a ratio of an area of the contact surface 11 of the cold compress member 1 to the total area of a vertical projection of all the electrodes on a reference plane which the contact surface 11 of the cold compress member 1 is located on is in a range from 3.5:1 to 5:1. For example, when the ratio is 5:1, the skin temperature may be maintained in a range from 38°C to 43°C, thereby providing more comfortable touch. When the ratio is in the range from 3.5:1 to 5:1, it is possible to ensure that the one or more electrodes 4 and the cold compress member 1 may contact the biological parts fully, and the good conductivity of the cold compress member 1 may also be performed. In this way, the temperature of the biological parts in contact with a contact head 1 may be maintained in a range from 38°C to 43°C, and the epidermis of the biological part may be maintained in a comfortable temperature range continuously.

If an area ratio is not properly designed, it may directly lead to poor heat dissipation effect, thereby leading to thermal pain in the biological parts, the feel freezing in the biological parts due to a good heat dissipation, or uneven temperature that one part is hot and another part is cold at the same time.

Optionally, the refrigeration member 2 may be made of pato stone or crystal.

Optionally, the heat dissipation member 3 may be a ceramic base. The ceramic base has a good thermal conductivity, which may improve the heat dissipation effect of the heat dissipation member 3.

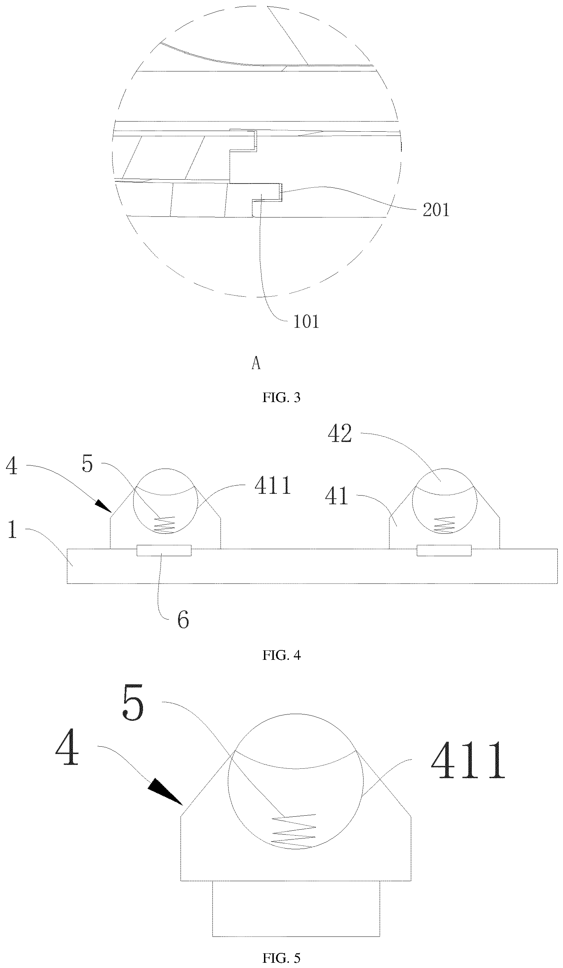

As shown in FIG. 4 and FIG. 5, optionally, an installation port 411 is defined on the fixing portion 41. A diameter of a projection of the installation port 411 on a plane which the cold compress member 1 is located on is smaller than that of a projection of the main body portion 42 on the plane which the cold compress 1 is located on. The main body portion 42 is at least partially exposed from the installation port 411. The main body portion 42 is effectively prevented from falling out by means of limiting the diameter of the installation opening 411, such that the main body portion 42 may be stably rolled on the fixing portion 41. At the same time, sufficient rolling space may be provided for the main body portion 42 via the installation opening 411, such that the main body portion 42 may roll smoothly, thus the main body portion 42 better roll with the biological parts, thereby reducing the pulling of the biological part and improving the convenience of using the beauty device.

Optionally, the main body portion 42 may be a sphere or a ball. The sphere has an advantage of being easy to roll. At the same time, the touch of the sphere is soft. When the sphere is in contact with the biological parts, the scratch on the biological parts may be reduced by the sphere, such that the beauty of the skin by the main body portion 42 may be ensured, and at the same time, the safety may be improved as much as possible. Optionally, the main body portion 42 is a conductor, that is, the main body portion 42 may discharge the biological parts, so as to achieve the effect of beautifying the biological part.

As shown in FIGS 2-5, optionally, the skin care assembly includes an elastic member 5. The elastic member 5 is arranged in the fixing portion 41 and located between the main body portion 42 and a bottom surface of the fixing portion 41. One end of the elastic member 5 abuts against the bottom surface of the fixing portion 41, and the other end of the elastic member 5 abuts against the main body portion 42, the main body portion 42 is pressed against the installation port 411 under an action of the elastic member 5.

When the biological parts are beautified by the one or more electrodes 4, the main body portion 42 may be contacted with the biological parts softly in a rolling manner via the elastic member 5, such that the elastic member 5 abuts against the main body portion 42, thus the main body portion 42 may be contacted with the biological parts fully, thereby improving beauty effect. By setting the elastic member 5, the main body portion 42 may be adjusted according to different biological parts during the process of contacting the biological parts, such that the main body portion 42 may roll better, thereby improving he convenience of using the beauty device.

Optionally, the elastic member 5 may be a spring or a soft silicone. In some embodiments of the present disclosure, the elastic member 5 is a spring, which is shown for illustration.

Optionally, the main body portion 42, the elastic member 5, and the fixing portion 41 are all conductors. When the beauty device is in operation, the main body portion 42 is configured for beautifying the biological parts, and in the process of beautifying the biological parts, the elastic member 5 and the main body portion 42 are always kept in abutment state, which is beneficial to conduct the current among the main body portion 42, the elastic part 5, and the fixing portion 41, such that the main body portion 42 discharges the biological parts, such that the biological parts may be better beautified, thereby improving the beauty effect.

Optionally, the skin care assembly include a vibrating generator 6. The each of the one or more electrodes 4 vibrates at a certain frequency under an action of the vibrating generator 6. The vibrating generator 6 may be installed between the fixing portion 41 and the cold compress member 1. An output terminal of the vibrating generator 6 is connected to the fixing portion 41, and the each of the one or more electrodes 4 vibrates at the certain frequency under the action of the vibrating generator 6. When the beauty device is in operation, the each of the one or more electrodes 4 may be maintained in a state of discharging the biological parts, and the each of the one or more electrodes 4 vibrates at the certain frequency so as to promote the blood circulation of the biological parts, thereby improving a state of the biological parts, activating the vitality, and enhancing the beauty effect.

As shown in FIGS. 1-3, the operation head of the skin care assembly may be provided in some embodiments of the present disclosure. The skin care assembly includes the support 70 and the shell 20. The shell 20 is in a shape of long strip, and defines a cavity inside. The support 70 is configured to install an internal structure of the beauty device into the shell 20, that is, the internal structure of the beauty device may be assembled in the shell 20 so as to utilize the space inside the shell 20. In this way, the space utilization inside the shell 20 is greatly improved. By setting the support 70, the internal structure of the beauty device may be fixed in the shell 20 to form a whole beauty device.

Optionally, the support 70 is fixed with the shell 20 by snapping or screws.

In some embodiments of the present disclosure, the support 70 is fixed with the shell 20 by snapping, which is shown for illustration. Optionally, the support 70 is arranged with a first fastener 101. The shell 20 is arranged with a second fastener 201. The support 70 fixed with the shell 20 by snapping the first fastener 101 and the second fastener 201.

Optionally, the first fastener 101 may be a cantilever hook or a fixing hole. In some embodiments of the present disclosure, the first fastener 101 is the cantilever hook. Optionally, the second fastener 201 may be also the cantilever hook or the fixing hole. In some embodiments of the present disclosure, the second fastener 201 is the fixing hole.

In some embodiments of the present disclosure, differing from the related art, the following technical effects may be achieved. The skin care assembly and the operation head of the skin care assembly are provided by some embodiments of the present disclosure. The cold compress member 1 includes the contact surface 11 and the conduction surface 12. The contact surface 11 is configured to contact the biological parts so as to cool and beautify the biological parts. The cooling side 21 of the refrigeration member 2 is thermally coupled with the conduction surface 12 of the cold compress member 1, thereby cooling the cold compress member 1. The heat dissipation member 3 is thermally coupled with the heat generating side 22 of the refrigeration member 2 to dissipate heat generated by the refrigeration member 2. The main body portion 42 is installed on the fixing portion 41 in the rolling manner. The fixing portion 41 is relatively fixed with the cold compress member 1 to fix the each of the one or more electrodes 4 on the refrigeration member 2. During the process of beatifying the biological parts, the each of the one or more electrodes 4 is contacted with the biological parts in a rolling manner via the main body portion 42, such that the friction between the each of the one or more electrodes 4 and the biological parts may be reduced, and the each of the one or more electrodes 4 may slide smoothly on the biological parts, thereby reducing a problem of pulling the skin or scratching the skin, and thus it is easy to use and simple to operate, which improves the convenience of using the beauty device during the use of the beauty device.

Optionally, a recess is defined on the fixing portion 41. The main body portion 42 is installed in the recess in a rolling manner and is not separated from the recess. An edge of the recess may be rounded to avoid cutting the skin.

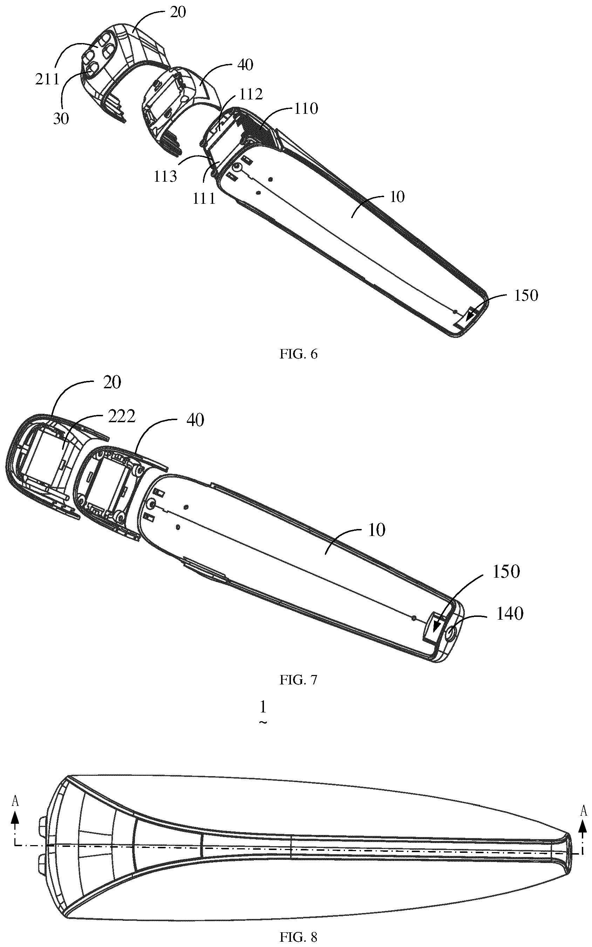

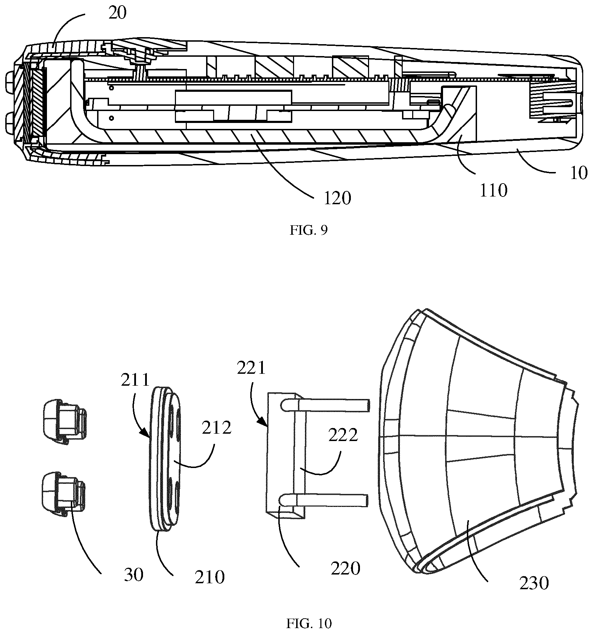

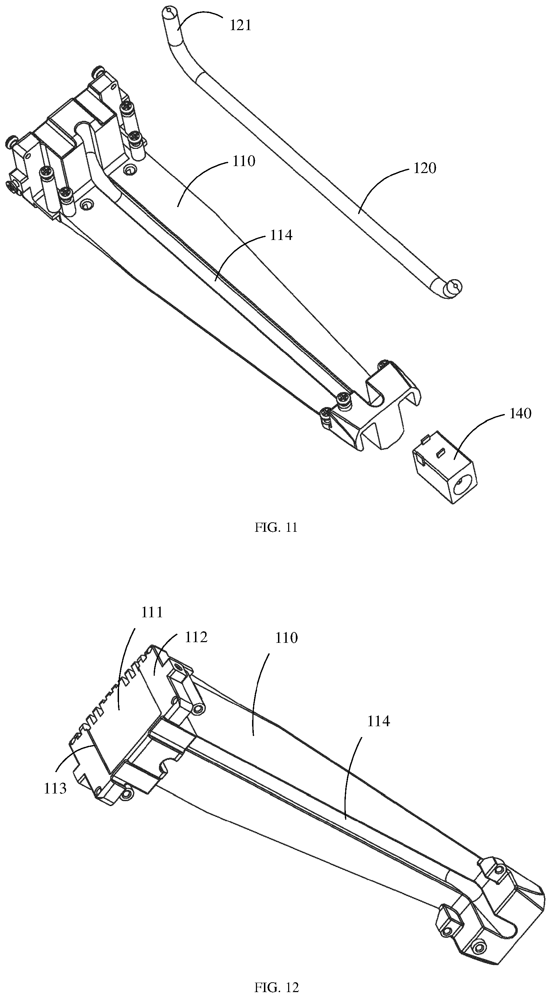

FIG. 6 is an exploded schematic view in a direction of the skin care assembly according to an embodiment of the present disclosure. FIG. 7 is an exploded schematic view in another direction of the skin care assembly as shown in FIG. 6. FIG. 8 is a structural schematic view of the skin care assembly according to an embodiment of the present disclosure. FIG. 9 is a cross-sectional view of the skin care assembly along A-A direction shown in FIG. 8. FIG. 10 is an exploded schematic view of an operation head according to an embodiment of the present disclosure. FIG. 11 is a structural schematic view of a heat dissipation member and a heat pipe according to an embodiment of the present disclosure. FIG. 12 is a structural schematic view in a direction of the heat dissipation member as shown in FIG. 11.

As shown in FIGS. 6-9, a device body 10 of the beauty device 1 may be provided in some embodiments of the present disclosure. The device body 10 includes a heat dissipation member 110. The heat dissipation member 110 includes a thermal coupling surface 111. Thermal coupling surface 111 is configured to be thermally coupled with a heat generating surface 222 of an operation head 20 so as to dissipate heat generated by the operation head 20. The operation head 20 includes a contact surface 211 and the heat generating surface 222. The contact surface 211 is configured to contact and cool the skin. The heat dissipation member 110 extends from a head end of the device body 10 to a trailing end of the device body 10.

Optionally, the beauty device 1 includes the device body 10, the operation head 20, and a second shell 40. The operation head 20 is installed on the device body 10 via the second shell 40.

FIG. 10 is an exploded schematic view of the operation head according to an embodiment of the present disclosure.

As shown in FIGS. 6-10, the operation head 20 may include an electrode head 30. The beauty device 1 may discharge the skin via the electrode head 30, such that the skin may be beautified by the following method, such as the radio frequency, the micro-current, or the like. The temperature of the skin may be increased during the discharge process of the electrode head 30. The operation head 20 further includes a first shell 230 fixed relatively to the electrode head 30.

In the device body 10 of the beauty device 1, the operation head 20 is dissipated heat via the heat dissipation member 110. The heat dissipation member 110 extends from the head end of the device body 10 to the trailing end of the device body 10, such that the heat dissipation member 110 may be enough long to increase a heat conduction path during heat dissipation. In this way, a longer heat conduction path may effectively prevent the heat from backtracking to the head end in a short time, thereby reducing the occurrence of heat backtracking.

At the same time, extending a heat dissipation path may improving the working efficiency of the operation head 20, which may meet the heat dissipation requirements in a case that rapid cooling is required. Reducing the heat backtracking may also prolong the cooling time of the contact surface 211 of the operating head 20. Further, the heating and cooling of the skin may be synchronized and balanced via the beauty device 1.

In some embodiments of the present disclosure, the device body 10 of the beauty device 1 may be applied to the skin care assembly, and may be also applied to a non-contact-type beauty device. When the device body 10 is applied to the skin care assembly, the contact surface 211 of the operation head 20 may contact and cool the skin. When the operation head 20 contacts and cools the skin, the heat on the skin may be transferred to the beauty device 1 through the contact surface 211. From a macroscopic point of view, a case that the heat is introduced into the beauty device 1 satisfies a general rule of transferring heat, that is, the heat is spontaneously transferred from an area with a high temperature to an area with a low temperature.

In the device body 10 provided by some embodiments of the present disclosure, when the temperature of the skin is higher than that of the contact surface 211 of the operation head 20, the heat transfers from the skin, and then passes through the operation head 20, the heat dissipation member 110, and so on. When a length of the heat dissipation member 110 is greater, the heat is transferred from the area with a high temperature to the area with a low temperature, such that the longer the conduction path needs to be passed, the more time it takes.

In the device body 10 of the beauty device 1 or the beauty device 1 to which it is applied, the heat is generally transferred from the area with a high temperature to the area with a low temperature. When the temperature of the device body 10 of the beauty device 1 or the parts of the beauty device 1 to which it is applied, especially the temperature of the heat dissipation member 110 is substantially equal to or close to the temperature of the skin, a case of the heat backtracking may occur. That is, the heat accumulated on the heat dissipation member 110 cannot be dissipated, and the heat is reversely transferred to the operating head 20, such that the temperature of the skin cannot be reduced, or the efficiency of reducing temperature is low. At this time, the beauty device 1 cannot achieve the beauty effect, or may cause negative effects such as tingling on the skin. By setting a longer heat dissipation member 110 may slow down the time for the occurrence of heat retrospection to a certain degree, and prolong the working time of the beauty device 1. In addition, the heating and cooling of the skin by the beauty device 1 may reach a balanced state.

Optionally, the length of the heat dissipation member 110 cannot be increased indefinitely, and the length may be synthetically designed in conjunction with indicators and sizes of each part of the device body 10 of the beauty device 1. For example, in the device body 10 of the beauty device 1 provided by the present disclosure, the heat dissipation member 110 extends from the head end of the device body 10 to the tail end of the device body 10.

Optionally, the heat dissipation member 110 is disposed on a trailing end of the device body 10, and a gap is defined between an ending of the heat dissipation member 110 and an ending of the trailing end of the device body 10. A size of the gap is less than one third of a length of the device body 10.

As discussed above, the length of the heat dissipation member 110 cannot be increased indefinitely, and the length may be synthetically designed in conjunction with the indicators and sizes of the each part of the device body 10 of the beauty device 1. A relationship between the length of the heat dissipation member 110 and the length of the device body 10 is provided by some embodiments of the present disclosure. Specifically, the heat dissipation member 110 is disposed on the trailing end of the device body 10, and the gap is defined between the ending of the heat dissipation member 110 and the ending of the trailing end of the device body 10. The size of the gap is less than one third of the length of the device body 10. Since the heat dissipation member 110 contacts the operation head 20 to transfer heat, and the gap is defined between the ending of the heat dissipation member 110 and the ending of the trailing end of the device body 10. At this time, a gravity center of the device body 10 may be close to the operation head 20. In some embodiments, the gravity center of the device body 10 may be in the middle of the device body 10, or may be close to the ending of the trailing end of the device body 10. A weight configuration of the device body 10 can be set as required.

During long-term research and exploration, the applicant has found that when the length of the heat dissipation member 110 is no more than that of the device body 10, and the gravity center of the heat dissipation member 110 is arranged close to the head, such that the user may grip more stably and comfortably during the use of the beauty device 1.

The gap is defined between the ending of the heat dissipation member 110 and the ending of the trailing end of the device body 10, such that a part of the space may be reserved for installing components that need to be installed on the trailing end of the device body 10, such as a charging port 140 and the like. A size of the gap may be less than one third of the device body 10. At this time, it cannot only meet the needs of the cooling time of the beauty device 1, but also facilitate the user to grasp and use the beauty device 1, and thus it is also possible to facilitate to fix some components, which serves three birds with one stone.

Optionally, in some embodiments, there is no gap between the heat dissipation member 110 and the ending of the trailing end of the device body 10. In some embodiments, the length of the heat dissipation member 110 is longer than that of the device body 10, such that the heat dissipation member 110 is exposed from the device body 10 or corresponding beauty device 1. The trailing end of the heat dissipation member 110 is directly exposed from the device body 10, which may be also possible to increase the heat dissipation efficiency and prolong the cooling time of the device body 10 or the beauty device 1.

Optionally, when the heat dissipation member 110 is exposed from the device body 10, an exposed part of the heat dissipation member 110 may be used with other components or devices so as to achieve some certain effects. For example, the exposed part of the heat dissipation member 110 may be used in conjunction with a cooling device, thereby further prolonging the cooling time or pre-cooling the heat dissipation member 110, which is convenient for customers to use. For example, the exposed part of the heat dissipation member 110 may be matched with a specific fixing mount so as to facilitate the placement of the beauty device 1. Compared with maintaining stability through the shell of the device body 10 when placed, the heat dissipation member 110 penetrates almost the entire beauty device 1, which is more convenient for maintaining stability. Other combinations and effects that may be obtained by simple derivation are all within the scope of the present disclosure, and will not be repeated here.

Optionally, an air outlet 150 is defined on the trailing end of the device body 10, and the trailing end of the heat dissipation member 110 is arranged corresponding to the air outlet 150.

The air outlet 150 is defined on the trailing end of the device body 10 so as to facilitate the device body 10 to dissipate heat. The trailing end of the heat dissipation member 110 is arranged corresponding to the air outlet 150, which may facilitate the heat conducted to the tail of the heat dissipation member 110 to dissipate from the air outlet 150. The heat in the device body 10 needs to dissipate into the air eventually to complete the heat dissipation. In this way, the air outlet 150 communicates the air inside and outside the device body 10, and cooperates with the heat dissipation member 110 extending to the trailing end, thereby effectively dissipating heat to the device main body 10.

Optionally, thermal coupling surface 111 is disposed on an end surface 112 of the head end of the heat dissipation member 110. A recess 113 is defined on the end surface 112. A bottom surface of the recess 113 is thermal coupling surface 111. The operation head 20 includes a cold compress member 210 and a refrigeration member 220. The cold compress member 210 includes a contact surface 211 and a conduction surface 212. A cooling side 221 of the refrigeration member 220 is thermally coupled with the conduction surface 212 of the cold compress member 210, so as to cool the cold compress member 210. A heat generating side of the refrigeration member 220 is a heat generating surface 222. The heat generating surface 222 is thermally coupled with the heat dissipation member 110, so as to dissipate heat generated by the refrigeration member 220. The refrigeration member 220 is disposed in the recess 113, and the cooling side 221 is higher than the recess 113.

Thermal coupling surface 111 is disposed on the end surface 112 of the head end of the heat dissipation member 110, and is thermally coupled with the heat generating surface 222 of the operation head 20, so as to dissipate the heat generated by the operation head 20. The recess 113 is disposed on the end surface 112, such that it is convenient to place and fix the refrigeration member 220, thereby improving the stability of the device body 10. A position of the refrigeration member 220 in a plane direction which the end surface 112 is located on may be limited by the recess 113, such that a position of the refrigeration member 220 is fixed in the plane direction. The refrigeration member 220 includes a cooling side 221 and a heat generating side. The cooling side 221 is configured to cool the cold compress member 210 and the like. The heat generating side generates heat due to the operation of the refrigeration member 220, and the heat is not beneficial to the operation of the device body 10 or the beauty device 1. In this case, it is necessary to arrange the heat dissipation member 110 to dissipate the heat of the heat generating side. The heat generating surface 222 of the operating head 20 is the heat generating side of the refrigeration member 220, and is thermally coupled with thermal coupling surface 111 of the refrigeration member 110. The cooling side 221 and the heat generating side are two sides of the refrigeration member 220. The heat dissipation member 110 dissipates the heat of the refrigeration member 220. Therefore, the heat of the heat generating side of the refrigeration member 220 may be conducted toe the heat dissipation member 110, such that the temperature of an area close to the refrigeration member 220 is higher than that of the cooling side 221 of the refrigeration member 220. According to the second law of thermodynamics, heat always flows spontaneously from an area with a high temperature to an area with a low temperature. Therefore, when the heat dissipation member 110 contacts the cooling side 221 of the refrigeration member 220, the heat that should be conducted out may be re-transferred to the cooling side 221 of the refrigeration member 220, resulting in backtracking of the heat, thereby affecting the cooling effect. In some embodiment, the cooling side 221 is higher than the recess 113, thereby preventing the cooling side 221 from contacting the heat dissipation member 110, and thus it is possible to reduce the heat backtracking and prolong the cooling time of the beauty device 1. The cooling time of the beauty instrument 1 is prolonged in a structural manner, such that it is possible to reduce the cooling burden of the refrigeration member 220 and reduce the related material and design costs. At the same time, it is also possible to improve the working efficiency of the refrigeration member 220, which may meet the heat dissipation requirements of the refrigeration member 220 in a case that rapid cooling is required.

Optionally, the heat generating side of the refrigeration member 220 may be a plane, and thermal coupling surface 111 may be also a plane. The heat generating side of the refrigeration member 220 is thermally coupled with thermal coupling surface 111 of the heat dissipation member 110, such that the heat generating side contacts thermal coupling surface 111 to transfer the heat. Alternatively, materials such as thermally conductive silicone grease or the like may be filled between the refrigeration member 220 and the heat dissipation member 110 so as to transfer the heat. The heat generating side of the refrigeration member 220 may directly or indirectly contact with thermal coupling surface 111, for example, a thermal conductive medium such as thermal conductive silicone grease is filled between the heat generating side of the refrigeration member 220 and thermal coupling surface 111.

FIG. 11 is a structural schematic view of a heat dissipation member and a heat pipe according to another embodiment of the present disclosure.

Optionally, a ratio of a thickness of the refrigeration member 220 to a depth of the recess 113 is in a range from 2 to 10. A unit of thickness of the refrigeration member 220 is millimeters, and a unit of depth of the recess 113 is millimeters.

The heat generating side of the refrigeration member 220 is thermally coupled with thermal coupling surface 111. The refrigeration member 220 is disposed in the recess 113, so as to limit a position of the refrigeration member 220 on the same plane as thermal coupling surface 111. In this way, the refrigeration member 220 may be relatively fixed with the heat dissipation member 110 without sliding, such that it is helpful for the stability of the structure of the beauty device 1, and the stable structure is helpful for dissipating heat. Two objects in contact with each other do not change their positions, and the heat is transferred smoothly and continuously from the area with high temperature to the area with low temperature. In this way, the efficiency of transferring heat is high and stable. During long-term research and exploration, the inventor has found that the ratio of the thickness of the refrigeration member 220 to the depth of the recess 113 is in a range from 2 to 10, which may not only meet the requirements of structural stability, but also ensure a good performance of the transferring heat, and it is further possible to prevent the heat from backtracking to the cooling side 221. The ratio of the thickness of the refrigeration member 220 to the depth of the recess 113 is in a range from 2 to 10, which may adapt to a size of the handheld beauty device, and the refrigeration member 220 in this ratio is stably disposed on the heat dissipation member 110.

Optionally, an installation method of the refrigeration member 220 is to be pressed into the recess 113 by a certain pressure, so as to save the cost of processing the refrigeration member 220 and corresponding installation structures. The ratio of the thickness of the cooling element 220 to the depth of the recess 113 is in a range from 2 to 10, such that the refrigeration member 220 is not easily to move relative to each other when the refrigeration member 220 is pressed into the recess 113. Even if the beauty device 1 falls off the ground or collides in daily use, the structure of the beauty device 1 may be kept stable. The refrigeration member 220 cannot pop out of the recess 113 and be stuck on an edge of the recess 113 due to the deformation caused by such a degree of collision. Therefore, the beauty device 1 has a better durability in the daily use.

Optionally, a section of a heat pipe 120 is bent to form a bending portion 121, the bending portion 121 abuts against an end of the heat dissipation member 110 close to the operation head 20. The heat pipe 120 has a hollow and sealing structure and is filled with liquid, and the liquid is not full of the heat pipe 120.

The heat pipe 120 is configured to accelerate the heat dissipation from the head end to the trailing end of the heat dissipation member 110. The section of the heat pipe 120 is bent to form the bending portion 121, the bending portion 121 abuts against the end of the heat dissipation member 110 close to the operation head 20, such that it is possible to increase an area of the heat pipe 120 close to the operation head 20, thereby improving the heat dissipation efficiency. When the heat is transferred from the operating head 20 to the heat dissipation member 110, and then transferred from the heat dissipation member 110 to the bending portion 121 of the heat tube 120. Therefore, there is also thermal coupling between the operation head 20 and the bending portion 121, and the bending portion 121 may improve the heat dissipation efficiency relative to the straight pipe or the like.

Optionally, an avoidance groove 114 is defined on the heat dissipation member 110, the heat pipe 120 is arranged along an inner wall of the avoidance groove 114 and welded to the inner wall of the avoidance groove 114, and an end of the heat pipe 120 away from the bending portion 121 extends to the end of the heat dissipation member 110.

The heat pipe 120 is disposed in the avoidance groove 114, and arranged along the inner wall of the avoidance groove 114, thereby increasing the contact surface 211 between the heat pipe 120 and the heat dissipation member 110. In this way, the heat pipe 120 may effectively conduct heat conduction to the heat dissipation member 110, thereby effectively helping the heat dissipation member 110 to quickly transfer the heat of the operation head 20 to the trailing end of the beauty device 1.

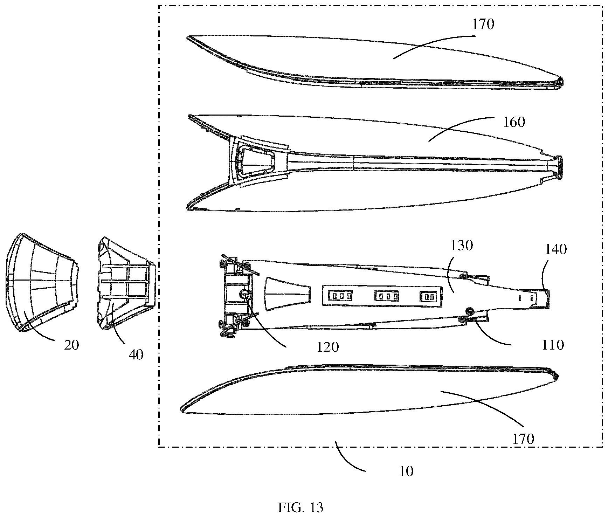

FIG. 13 is an exploded schematic view in another direction of a beauty device according to an embodiment of the present disclosure.

Optionally, as shown in FIG. 13, the device body 10 further includes an installation plate 130 and the charging port 140. The installation plate 130 is installed on the opening of the recess 113 and extends towards the trailing end of the device body 10. The charging port 140 is disposed on an end of the installation plate 130 and exposed from the device body 10. The installation plate 130 is configured to install necessary components or elements of the beauty device 1, such as a circuit board, a battery, and so on. The beauty device 1 may be used by plugging in or by charging. When he beauty device 1 is used by charging, the battery is charged by the beauty device 1 by means of the charging port 140 being connected with an external power cord. When used by plugging, the beauty device 1 is used by means of the charging port 140 being connected with the external power cord. When the beauty device 1 is used by plugging, a certain impedance may be generated by the charging port 140 due to the case that internal points of the charging port 140 contact, such that the heat may be generated when the current passes. Therefore, the charging port 140 is disposed on an ending of the installation plate 130 and away from the heat dissipation member 110, such that it is possible to prevent the heat generated by the charging port 140 from being traced back to the device body 10 and even the operation head 20, which may affect the cooling effect, thereby affecting the beauty effect.

Optionally, the heat dissipation member 110 is integrally formed.

The heat dissipation member 110 may be processed by an entire piece of aluminum, so as to obtain a structure required for partial installation, supporting and keeping space. The integrally formed heat dissipation member 110 has better integrity, and each structure is more coherent, which is conducive to dissipate heat. When heat flows through one object and two objects in contact with each other, the heat conduction efficiency of the former is better than that of the latter.

Optionally, the beauty device body further includes a third shell 160 and an appearance member 170. The heat dissipation member 110 and the charging port 140 are installed and received in the third shell 160. The number of the appearance members 170 may be two, and the two appearance members 170 are disposed outside of the third shell 160. The appearance member 170 may be an elastic, high damping material such as silicone, sponge, or the like. The appearance member 170 made of the elastic and the high damping material may provide a more comfortable and secure grip for the user.

The skin care assembly may also be provided in some embodiments of the present disclosure. The skin care assembly includes the above-mentioned device body 10 of the beauty device. The beauty device further includes the operation head 20. The operation head 20 includes the contact surface 211 and the heat generating surface 222. The contact surface 211 is configured to contact and cool the skin. The operation head 20 is disposed on the head end of the skin care assembly.

In some embodiments of the present disclosure, the following technical effects of the device body of the beauty device may be achieved.

In some embodiments of the present disclosure, in the device body 10 of the beauty device 1, heat of the operation head 20 is dissipated via the heat dissipation member 110. The heat dissipation member 110 extends from the head end of the device body 10 to the trailing end of the device body 10, such that the heat dissipation member 110 may be enough long to increase a heat conduction path during heat dissipation. In this way, a longer heat conduction path may effectively prevent the heat from backtracking to the head end in a short time, thereby prolonging the cooling time of the contact surface 211 of the operating head 20. The heat dissipation member 110 is disposed on the trailing end of the device body 10, and the gap is defined between the ending of the ending of the heat dissipation member 110 and the ending of the trailing end of the device body 10. A size of the gap is less than one third of a length of the device body 10. In this way, the heat dissipation member 110 may not only meet needs of heat dissipation, but also make the center of gravity of the beauty device 1 meet the needs of use.

The skin care assembly may also be provided in some embodiments of the present disclosure. The skin care assembly includes the above-mentioned device body 10 of the beauty device 1. The beauty device 1 further includes the operation head 20. The operation head 20 includes the contact surface 211 and the heat generating surface 222. The contact surface 211 is configured to contact and cool the skin. The operation head 20 is disposed on the head end of the skin care assembly. In some embodiments of the present disclosure, the sapphire may be either artificial or natural.

The skin care assembly may also be provided in some embodiments of the present disclosure. The skin care assembly includes the above-mentioned device body of the beauty device. The beauty device further includes the operation head. The operation head includes a contact surface and a heat generating surface. The contact surface is configured to contact and cool the skin. The operation head is disposed on the head end of the skin care assembly.

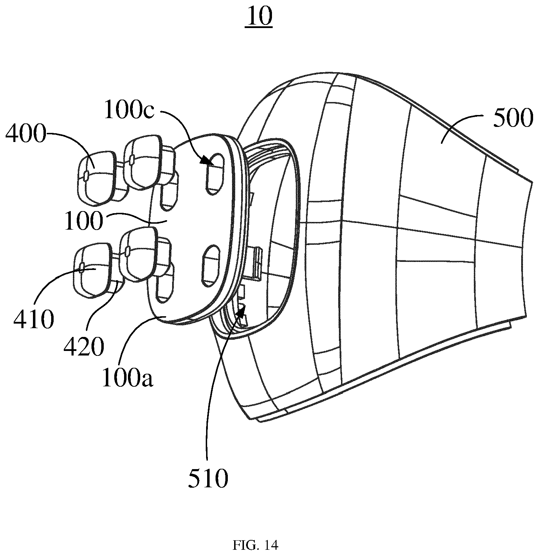

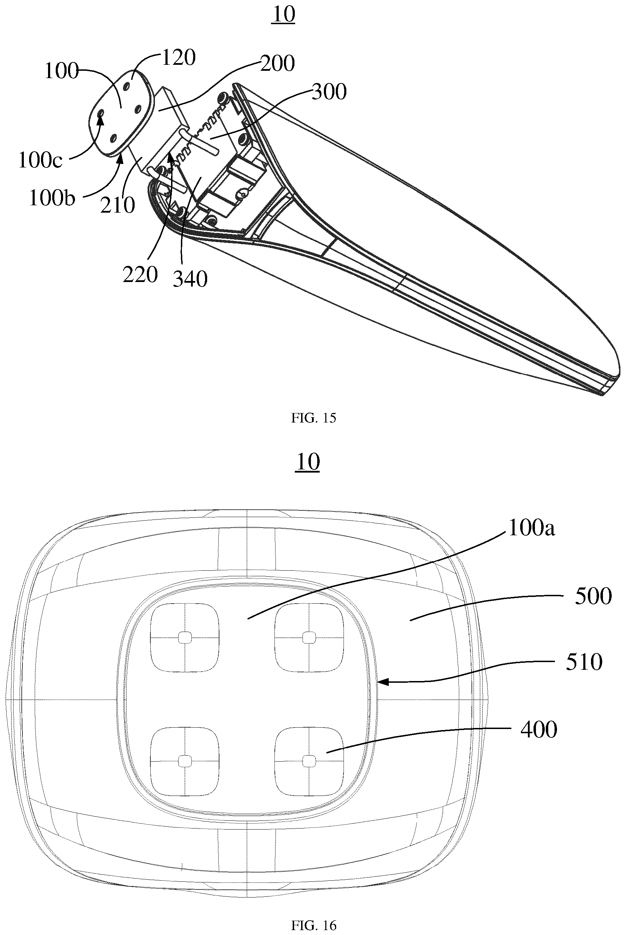

FIG. 14 is a perspective exploded schematic view of the skin care assembly according to an embodiment of the present disclosure. FIG. 15 is a perspective exploded schematic view of the skin care assembly according to another embodiment of the present disclosure.

As shown in FIG. 14 and FIG. 15, the skin care assembly 10 includes a cold compress member 100, a refrigeration member 200, a heat dissipation member 300, one or more electrode heads 400, and a first cover 500. The cold compress member 100 includes a contact surface 100a and a conduction surface 100b. The contact surface 100a is configured to contact the skin. The cooling side 210 of the refrigeration member 200 is thermally coupled with the conduction surface 100b of the cold compress member 100, thereby cooling the cold compress member 100. The heat dissipation member 300 is thermally coupled with the heat generating side 220 of the refrigeration member 200 to dissipate heat generated by the refrigeration member 200. The each of the one or more electrode heads 400 is relatively fixed with the cold compress member 100, and is configured to discharge and heat the contacted skin. The first cover 500 is configured to fix the cold compress member 100 and the refrigeration member 200 to the heat dissipation member 300. Optionally, the cold compress member 100 and the one or more electrode heads 400 may be combined with other parts of the skin care assembly 10 via the first cover 500, thereby forming a complete skin care assembly. In this way, the cold compress member 100 and the refrigeration member 200 are fixed to the heat dissipation member 300 via the first cover body 500, which is also more convenient during assembly and installation.

When the skin care assembly 10 is in operation, the one or more electrodes 4 are discharged, for example, to generate the radio frequency or the micro-current. The current passes through and stimulates the skin, such that the desired beauty effect may be achieved. Since the skin is equivalent to the electrical resistance, a temperature of the skin may increase. The cold compress member 100, the refrigeration member 200, and the heat dissipation member 300 are thermally coupled with each other, such that the heat generated by the skin may be conducted from the cold compress member 100to the heat dissipation member 300 via the refrigeration member 200, thereby dissipating the heat from the skin care assembly.

Optionally, the cold compress member 100is relatively fixed with the each of the one or more electrode heads 400. The refrigeration member 200 is disposed between the heat dissipation member 300 and the cold compress member 100 to form a sandwich structure of the heat dissipation member 300, the refrigeration member 200, and the cold compress member 100. Optionally, the first cover 500 is configured to fix the cold compress member 100 and the refrigeration member 200 in the heat dissipation member 300. The cold compress member 100 and the one or more electrode heads 400 contacted the skin, and the cold compress member 100 is adjacent to the each of the one or more electrode heads 400. On the one hand, when the skin located deeper is discharged and heated by the one or more electrode heads 400, at the same time, the cold compress member 100 may provide a lower temperature environment via the refrigeration member 200, such that the skin located shallower is cooled by the cold compress member 100 in time, thereby improving touch for the biological parts. On the other hand, the cold compress member 100 and the refrigeration member 200 are fixed in the heat dissipation member 300 via the first cover 500, such that it is possible to facilitate the assembly and installation of the skin care assembly. For example, when the cold compress member 100 or the cooling element 200 in the skin care assembly needs to be replaced, it is only necessary to take out the first cover 500 from the heat dissipation member 300, and the cold compress element 100 and the cooling element 200 may be removed from the skin care assembly and replaced. During installation, the cold compress element 100 and the refrigeration member 200 are sequentially fixed on the heat dissipation member 300 via the first cover 500, which is simple and convenient.

A muscle contraction movement is caused when the current passes through the skin, thereby achieving the beauty effect. During a discharge process of the one or more electrode heads 400, the skin temperature and the dermal temperature increase. The skin temperature is increased due to heat generated by skin cells and heat converted from electrical energy. The heat generated by the one or more electrode heads 400 stimulating the epidermis is reduced via the cold compress member 100, thereby cooling the skin epidermis, and achieving a cold compress and cooling function. In this way, it is possible to reduce the situation that the skin is burned due to excessive temperature, thereby reducing the discomfort during use and improving the operating comfort of the operation head. The cold compress member 100 is cooled by the refrigeration member 200, such that the skin epidermis may be continuously cooled by the cold compress member 100. During the process of alternating hot and cold, it is also possible to expand contract the skin, such that the skin's breathing is enhanced, thereby protecting the skin from damage and better stimulating the tightening and regeneration of dermal collagen, tightening the skin, and improving beauty effect.

Optionally, the skin may be facial skin, arm skin, or the like. The skin located shallower may be the epidermis, and the skin located deeper may be the dermis. When the one or more electrode heads 400 is in operation, the discharge may generate the radio frequency, the microcurrent, or the like, so as to beautify the skin.

The heat generated on the skin by the one or more electrode heads 400 is conducted to the cold compress member 100, and the skin is contacted with the contact surface 100a of the cold compress member 100, such that the epidermis is cooled by the cold compress member 100. The conduction surface 100b of the cold compress member 100 is thermally coupled with the cooling side 210 of the refrigeration member 200, such that the cold compress member 100 is cooled by the refrigeration member 200 to absorb the heat conducted by the cold compress member 100. When the refrigeration member 200 is in operation, the heat may be generated on the heat generating side 220 accordingly, and the heat generating side 220 of the refrigeration member 200 is thermally coupled with the heat dissipation member 300, such that the heat generated by the refrigeration member 200 is conducted to the heat dissipation member 300 and passes through the heat dissipation member 300, and thus the heat may be exhausted from the skin care assembly via the heat dissipation member 300. During an entire process of transferring heat, the return of heat is reduced in a short duration, thereby prolonging the time that the skin care assembly maintains a state of the temperature balance.

Optionally, thermal coupling between the cooling side 210 of the refrigeration member 200 and the conduction surface 100b of the cold compress member 100, and thermal coupling between the heat dissipation member 300 and the heat generating side 220 of the refrigeration member 200 may be realized by means of heat transmission, thermal convection, thermal radiation, or any combination of the above-mentioned three methods.

As shown in FIG. 14 and FIG. 15, an opening 510 is defined on the first cover 500. Optionally, the opening 510 of the first cover 500 is arranged corresponding to the cold compress member 100. A size of the opening 510 in at least one radial direction is smaller than a size of the cold compress member 100 in the radial direction. An edge of the cold compress member 100 is pressed to the heat dissipation member 300 through an edge of the opening 510, such that the cold compress member 100 and the refrigeration member 200 are fixed on the heat dissipation member 300.

Optionally, a plane size of the contact surface 100a of the cold compress member 100 is substantially equal to that of the opening 510. A plane size of the conduction surface 100b of the cold compress member 100 is greater than that of the opening 510. In this way, when the conduction surface 100b of the cold compress member 100 is thermally coupled with the cooling side 210 of the refrigeration member 200, and the heat generating side 220 of the refrigeration member 200 is thermally coupled with the heat dissipation member 300, a side of the first cover 500 is disposed in the contact surface 100a of the cold compress member 100 via the opening 510. Therefore, an edge of the conduction surface 100b of the cold compress member 100 may be pressed towards the heat dissipation member 300 in an axial direction by the edge of the opening 510, such that the cold compress member 100 and the refrigeration member 200 are fixed in the heat dissipation member 300. In a design of the skin care assembly 10, the cold compress member 100 and the refrigeration member 200 have specific functions, and the manufacturing and processing processes are generally complicated. Therefore, the opening 510 is defined on the first cover 500, such that the cold compress member 100 and the refrigeration member 200 are fixed in the heat dissipation member 300 with a simple structure, thereby saving the internal space of the skin care component 10, reducing the production difficulty, and decreasing the production cost.

FIG. 16 is a front structural schematic view of the skin care assembly according to another embodiment of the present disclosure. Optionally, each of the one or more electrode heads 400 is disposed on the edge of the opening of the first cover 500. The number of the electrode heads 400 is at least two. In some embodiments, the at least two electrode heads 400 are arranged on an outer periphery of the contact surface 100a of the cold compress member 100 and spaced apart from each other.

Optionally, as shown in FIGS. 14-16, each of the one or more electrode heads 400 includes an end portion 410 and an installation post 420. An installation hole 100c is defined on the contact surface 100a of the cold compress member 100. The installation hole 100c is inserted through the entire cold compress member 100. A size of the installation hole 100c is set correspondingly to the installation post 420. The installation post 420 is inserted through the installation hole 100c, such that the one or more electrode heads 400 are installed on the cold compress member 100. The installation post 420 may be fixed relatively to the cold compress member 100 via an end of the installation post 420 close to the conduction surface 100b. For example, the end of the installation post 420 close to the conduction surface 100b is arranged with threads, and screw caps may be used to fix the one or more electrode heads 400 to the cold compress member 100. The one or more electrode heads 400 may also be kept relatively fixed to the cold compress member 100 by other means.

Optionally, the installation post 420 is inserted through the cold compress member 100, and the end portion 410 is installed on a surface of the contact surface 100a of the cold compress member 100. When the end portion 410 and the contact surface 100a contact the skin, and the end portion 410 starts to transmit electricity, the heat generated by the end portion 410 from the skin may be conducted radially to the surrounding via the contact surface 100a of the cold compress member 100 disposed around the end portion 410. In this way, the contact surface 11 of the cold compress member 100 may be designed to be small enough to save costs while ensuring the desired cooling effect without causing the large area of cold feeling at the parts in contact with the skin.

An installation relationship is between the each of the one or more electrode heads 400 and the cold compress member 100. Optionally, the each of the one or more electrode heads 400 installed on the cold compress member 100 may be distributed in the center of the cold compress member 100, or may be distributed along a circumference. The end portion 410 of the each of the one or more electrode heads 400 and the contact surface 100a of the cold compress member 100 contact with the skin. Since the one or more electrode heads 400 are installed an entire piece of the cold compress member 100, it is not necessary to install additional structures, such that there are no excess structures, such as a groove, a recess, a protrusion, an edge, or the like, to scratch the skin. Therefore, the one or more electrode heads 400 are installed on the entire piece of the cold compress member 100, which has comfort and heat transmission performances, thereby improving the comport of the parts in contact with the skin. When the skin care assembly 10 is in operation, end portion 410 of the each of the one or more electrode heads 400 and the contact surface 100a of the cold compress member 100 may stably contact the skin at the same time. Therefore, the each of the one or more electrode heads 400 is directly installed on the cold compress member 100, such that the skin care assembly 10 has a comfort and conduction performances.

Optionally, the skin care assembly 10 includes at least two electrode heads 400. The at least two electrode heads 400 are arranged on an outer periphery of the cold compress member 100 and spaced apart from each other. Optionally, at least one electrode head 400 is a negative electrode, and at least one electrode head 400 is a positive electrode. Optionally, the electrode heads 400 may be distributed on the cold compress member 100 in a point shape, or may be distributed on the cold compress member 100 in a line shape. The distribution of the electrode heads 400 cannot be too dense, and the spacing distribution makes the end portion 410 of each electrode head 400 contact with the skin, and the cold compress member 100 may also fully contact with the skin. It is possible to reduce the case that a distance between one end portion 410 of each electrode head 400 and other end portion 410 of each electrode head 400 is too small to block the contact surface 100a of the cold compress member 100, resulting in that the contact surface 100a of the old-compressing member 100 between the end portions 410 cannot contact the skin.

Optionally, one or more electrode heads 400 may be disposed on the cold compress member 100. The one or more electrode heads 400 are distributed along a circumference direction of a polygon and spaced apart from each other. When the cold compress member 100 is a square with arc chamfers, the four electrode heads 400 are respectively distributed at the four arc chamfers. The one or more electrode heads 400 may be distributed in a radial shape (such as a flower shape or a star shape) as a whole on the polygon. The one or more electrode heads 400 may also be distributed in a line shape or a plurality of line shapes on the polygon.

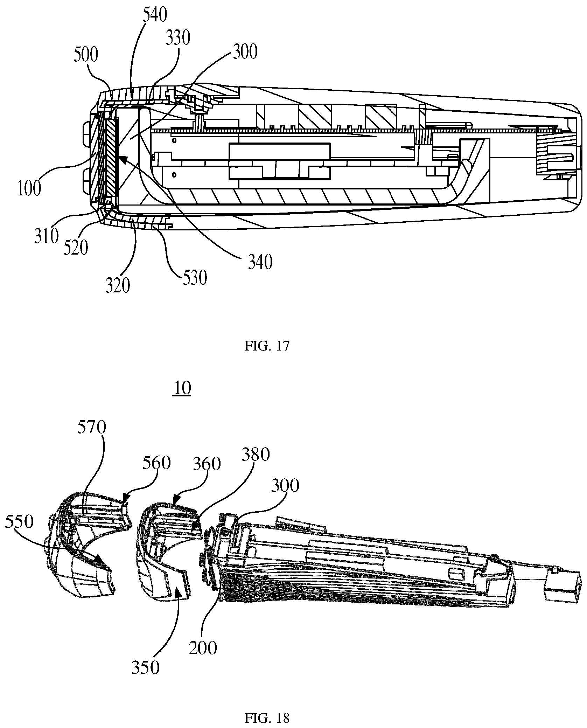

FIG. 17 is a cross-sectional structural schematic view of the skin care assembly according to another embodiment of the present disclosure.

Optionally, a first fastener 520 is fixed on the first cover 500. A second fastener 310 is fixed on the heat dissipation member 300 and snap-connected to the first fastener 520, such that the cold compress member 100 and the refrigeration member 200 are fixed on the heat dissipation member 300 via the first cover 500. Optionally, the fist cover 500 is a square cover including four side surfaces, and the first fastener 520 may be fixed on at least one side surface. The heat dissipation member 300 is also a square structure, and the second fastener 310 is disposed correspondingly to the first fastener 520 on a side surface of the heat dissipation member 300. The first fastener 520 is fastened into the second fastener 310, such that the fist cover 500 may be fixed with the heat dissipation member 300.

Optionally, each of a first side surface 530 and a second side surface 540 of the fist cover 500 is arranged with the first fastener 520. Each of a corresponding third side surface 320 and a corresponding fourth side surface 330 of the heat dissipation member 300 is correspondingly arranged with the second fastener 310. Further, the heat generating side 220 of the refrigeration member 200 is thermally coupled with the heat dissipation member 300. The cooling side 210 of the refrigeration member 200 is thermally coupled with the conduction surface 100b of the cold compress member 100. Optionally, the plane size of the contact surface 100a of the cold compress member 100 is substantially equal to that of the opening 510. The fist cover 500 is sleeved on the contact surface 100a via the opening 510, and the first fastener 520 is fastened into the second fastener 310, such that the first fastener 520 is locked by the second fastener 310 and the first cover 500 is pulled, thereby providing a force along an axial direction of the skin care assembly 10. In this way, the first cover 500 is fixed on the heat dissipation member 300, such that the cold compress member 100 and the refrigeration member 200 are fixed on the heat dissipation member 300 via the first cover 500.

Optionally, the first fastener 520 may be the cantilever hook. The second fastener 201 may be the fixing hole. By setting the cantilever hook and the fixing hole, the fixing hole is locked by the cantilever hook, such that the fist cover 500 is fixed on the heat dissipation member 300.

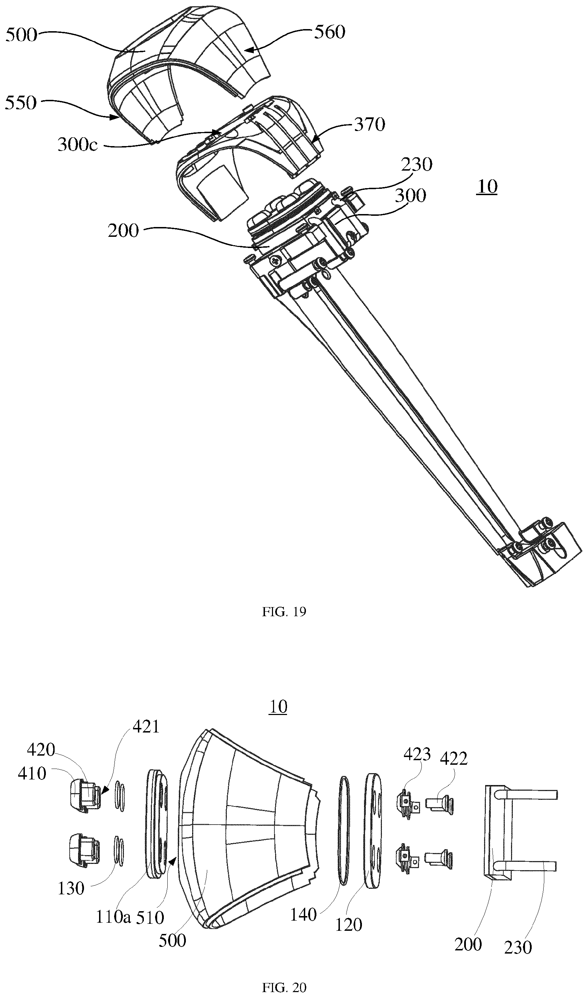

FIG. 18 is a perspective exploded structural schematic view of the skin care assembly according to some embodiments of the present disclosure viewed from a view angle. FIG. 19 is a perspective exploded structural schematic view of the skin care assembly according to some embodiments of the present disclosure viewed from another view angle.

Optionally, as shown in FIGS. 17-19, the heat dissipation member 300 includes a thermal coupling surface 340, and thermal coupling surface 340 is thermally coupled with the heat generating side 220 of the refrigeration member 200. Two sides of thermal coupling surface 340 are respectively connected with the third side surface 320 and the fourth side surface 330. A part of the second fastener 310 is disposed a side of thermal coupling surface 340 of the heat dissipation member 300 and snap-connected to the first fastener 520. A remaining part of the second fastener 320 extends towards the third side surface 320 and the fourth side surface 330 of the two sides of thermal coupling surface 340. A first guidance portion 350 and a second guidance portion 360 are arranged on an outside of the second fastener 310 and are configured to guide the first fastener 520 disposed on each of the first side surface 530 and the second surface 540 of the first fastener 520 to fasten into the second fastener 310. Optionally, the first side surface 530 and the second surface 540 of the first fastener 520 are arranged with a first lateral wing 550 and a second lateral wing 560. The first lateral wing 550 and the second lateral wing 560 are corresponding to the third side surface 320 and the fourth side surface 330 of the heat dissipation member 300. The first lateral wing 550 and the second lateral wing 560 of the first cover 500 are assembled with the heat dissipation member 300 via the first guidance portion 350 and the second guidance portion 360.

Optionally, the third side surface 320 and/or the fourth side surface 330 connected to thermal coupling surface 340 may be arranged with one or more second fasteners 310, which is not limited herein. Optionally, the first side surface 530 and the second surface 540 of the first fastener 520 are correspondingly arranged with the same number of the first fasteners 520. The first lateral wing 550 and the second lateral wing 560 of the first cover 500 are configured to guide the first fastener 520 fasten into the second fastener 310 via the first guidance portion 350 and the second guidance portion 360 correspondingly, such that it is more convenient to install and align when the skin care assembly 10 is assembled with the first cover 500 and the heat dissipation member 300.

Optionally, the first lateral wing 550 and/or the second lateral wing 560 of the first cover 500 are arranged with one of a guidance groove 370 and a guidance rib 570. One of the guidance groove 370 and the guidance rib 570 extends towards the third side surface 320 and/or the fourth side surface 330 of the heat dissipation member 300 along the first side surface 530 and/or the second surface 540 of the first cover 500. The third side surface 320 and/or the fourth side surface 330 of the heat dissipation member 300 are arranged with one of the guidance groove 370 and the guidance rib 570.

Optionally, the guidance rib 570 is assembled with the guidance groove 370 in a sliding manner, such that the guide of the guidance groove 370 or the guidance rib 570 disposed on the first lateral wing 550 and/or the second lateral wing 560 of the first cover 500 are guided by the corresponding guidance groove 370 or the guidance rib 570 of the first guidance portion 350 and the second guidance portion 360, thereby fastening the second fastener 310 into the first fastener 520.

Optionally, one of the two lateral wings of the first cover 500 and the corresponding one of the two side surfaces of the heat dissipation member 300 are smooth. The other one of the two lateral wings of the first cover 500 and the corresponding other one of the two side surfaces of the heat dissipation member 300 are arranged with the guidance groove 370 and the guidance rib 570 respectively. In this way, the first side surface 530 and the second surface 540 of the first cover 500, and the third side surface 320 and the fourth side surface 330 of the heat dissipation member 300 may be clearly distinguished, thereby playing a foolproof role.

Optionally, as shown in FIG. 18 and FIG. 19, the second lateral wing 560 of the second surface 540 of the first cover 500 is arranged with the guidance rib 570. The first lateral wing 550 of the first surface 530 is a smooth surface. The second guide portion 360 of the fourth side 330 of the of the heat dissipation member 300 is correspondingly arranged with the guidance groove 370. The first guidance portion 350 of the third side surface 320 is a smooth surface. Optionally, the first side surface 530 and the third side surface 320 are correspondingly arranged, such that the first fastener 520 of the first cover 500 may be smoothly fastened into the second fastener 310 of the heat dissipation member 300 via a cooperation of the guidance rib 570 and the guidance groove 370, thereby improving the convenience of the installation and use of the skin care assembly 10.

FIG. 20 is a partial exploded structural schematic view of the skin care assembly according to an embodiment of the present disclosure.

Optionally, the cold compress member 100 includes a cold compress sheet 110a and a cold-guiding sheet 110. The cold-guiding sheet 120 is disposed between the cold compress sheet 110a and the refrigeration member 200. The contact surface 100a is disposed a side of the cold compress sheet 110a away from the refrigeration member 200. The conduction surface 100b is disposed a side of the cold-guiding sheet 120 away from the cold compress sheet 110a. A size of the cold-guiding sheet 120 in at least one radial direction is greater than a size of the opening 510 of the first cover 500 in the radial direction, such that an edge of the cold-guiding sheet 120 is pressed to the heat dissipation member 300 by an edge of the opening 510 of the first cover 500.

Optionally, as shown in FIG. 20, the cold compress sheet 110a is disposed in one of an inner side and an outside of the opening 510, and the cold-guiding sheet 120 is disposed in the other of the inner side and the outside of the opening 510. A fixing hole 421 is defined on the installation post 420 of the electrode head 400 in an axial direction. The installation post 420 is inserted into the cold compress sheet 110a and the installation hole 100c of the cold-guiding sheet 120, and the installation post 420 is fixed by a fixing member 422. In this way, the electrode head 400 may be installed on the cold compress member 100, and the cold compress sheet 110a and the cold-guiding sheet 120 are fixed on the inner side and the outside of the openings 510. Optionally, the fixing hole 421 may be a threaded hole, and the fixing member 422 may be a screw. A size of the cold-guiding sheet 120 in a radial direction is greater than a size of the opening 510 of the first cover 500 in the radial direction, such that when the skin care assembly 10 is assembled, an edge of the cold-guiding sheet 120 is pressed to the heat dissipation member 300 by an edge of the opening 510 of the first cover 500, thus the cold compress member 100 and the refrigeration member 200 are fixed in the heat dissipation member 300.

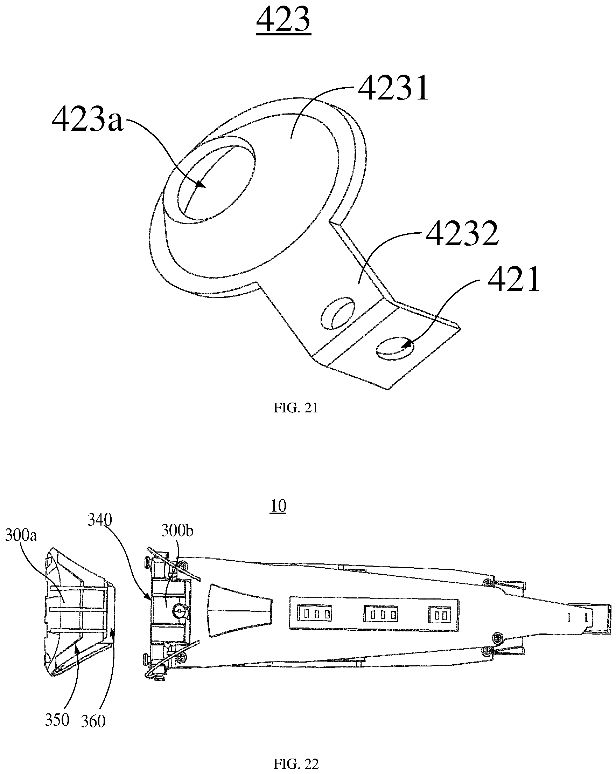

FIG. 21 is a partial exploded structural schematic view of a connection member of the skin care assembly according to another embodiment of the present disclosure.

Optionally, a connection member 423 is arranged between the cold-guiding sheet 120 and the fixing member 422, and is configured to fix the cold compress member 100 and the refrigeration member 200. The connection member 423 includes a main body portion 4231 and an extending portion 4232. A through hole 423a is defined on the main body portion 4231. The main body portion 4231 is inserted into the through hole 423a and the installation hole 100c via the fixing member 422, such that the connection member 423 is fixed to the cold compress member 100. Optionally, the extending portion 4232 is arranged with two right-angled sides substantially perpendicular with each other. The fixing hole 421 is defined on each of two side edges corresponding to the two right-angled sides, and the two side edges are connected to each other and substantially perpendicular with each other. In addition, the extending portion 4232 is inserted into the fixing hole 421 via the fixing member 422, such that the cold compress member 100 is fixed to the refrigeration member 200.

Optionally, the cold compress sheet 110a of the cold compress member 100 is contacted with the cooling side 210 of the refrigeration member 200 by the method of direct surface contact. Alternatively, the cold compress member 100 is contacted with the cooling side 210 by the method of indirect surface contact via the conduction surface 100b of the cold-guiding sheet 120. Therefore, by the method of the surface contact, the heat conduction is more sufficient, and the conduction effect may be maximized.

Optionally, as shown in FIG. 20, the cold-guiding sheet 120 may be aluminum nitride ceramics or other materials with good thermal conductivity. The heat transmission effect may be maximized by the method of the surface contact. The electrode heads 400, the cold compress sheet 110a, and the cold-guiding sheet 120 are made of materials with good heat transmission performance. However, when the electrode heads 400, the cold compress sheet 110a, and the cold-guiding sheet 120 are contacted with each other, gaps may be defined between the electrode heads 400, the cold compress sheet 110a, and the cold-guiding sheet 120. Optionally, a first sealing ring 130 is disposed between a gap defined between the electrode head 400 and the cold compress sheet 110a, and a second sealing ring 140 is disposed between a gap defined between the cold compress sheet 110a and the cold-guiding sheet 120, thereby filling the gaps between electrode head 400, the cold compress sheet 110a, and the cold-guiding sheet 120. For example, thermal grease may be filled in the gaps, which may make the heat transfer more sufficient, and also make the temperature control more smoothly, thereby improving the comfort of the skin feeling.

Optionally, the cold compress sheet 110a is disposed in the opening 510, and the cold compress sheet 110a has a first radial dimension and a second radial dimension different from the first radial dimension at different thickness positions. The opening has a third radial dimension and a fourth radial dimension different from the third radial dimension at different depth positions. Optionally, a part of the cold compress sheet 110a having the first radial dimension is closer to the cold-guiding sheet 120 than a part of the cold compress sheet 110a having the second radial dimension. A part of the opening 510 having the third radial dimension is closer to the cold-guiding sheet 120 than a part of the opening 510 having the fourth radial dimension. The first radial dimension is smaller than the third radial dimension, the second radial dimension is smaller than the fourth radial dimension, and the second radial dimension is greater than the third radial dimension.

Optionally, the cold compress sheet 110a is installed in the opening 510 from the outside of the opening 510. On the one hand, the cold compress sheet 110a having the first radial dimension is partially disposed in the opening 510 having the third radial dimension, and the first radial dimension is smaller than the third radial dimension. The second sealing ring 140 is filled in gaps defined in the part of the first radial dimension and the third radial dimension. In this way, the cold-guiding sheet 120 is disposed a side of the second sealing ring 140 away from the cold compress sheet 110a, such that the cold compress sheet 110a and the cold-guiding sheet 120 are firmly and hermetically fixed on the inner side and the outside of the opening 510. On the other hand, the cold compress sheet 110a having the second radial dimension is partially disposed in the opening 510 having the fourth radial dimension, and the second radial dimension is smaller than the fourth radial dimension. The first sealing ring 130 is filled in gaps defined in the part of the second radial dimension and the fourth radial dimension. In this way, the cold-guiding sheet 120 is completely disposed in the opening 510, and the first sealing ring 130 is disposed between the end portion 410 of each electrode head 400 and the cold compress sheet 110a, such that the electrode heads 400 and the cold compress sheet 110a are firmly and hermetically installed Optionally, a first area in the axial direction of the part of the first radial dimension of the cold compress sheet 110a in a thickness direction is greater than a second area in the axial direction of the part of the second radial dimension. A third area in the axial direction of the part of the third radial dimension of the opening 510 in a depth direction is greater than a fourth area in the axial direction of the part of the fourth radial dimension. The first area is substantially equal to the third area. The second area is substantially equal to the fourth area. Optionally, the part of the first radial dimension of the cold compress sheet 110a is greater than the part of the third radial dimension of the opening 510, such that the volume of the cold compress sheet 110a is large enough to conduct heat more sufficiently, thereby maximizing the conduction effect. On a side of the opening 510.

Optionally, a first area in the axial direction of the part of the first radial dimension of the cold compress sheet 110a in a thickness direction is greater than a second area in the axial direction of the part of the second radial dimension. A third area in the axial direction of the part of the third radial dimension of the opening 510 in a depth direction is greater than a fourth area in the axial direction of the part of the fourth radial dimension. The first area is substantially equal to the third area. The second area is substantially equal to the fourth area. Optionally, the part of the first radial dimension of the cold compress sheet 110a is greater than the part of the third radial dimension of the opening 510, such that the volume of the cold compress sheet 110a is large enough to conduct heat more sufficiently, thereby maximizing the conduction effect.

FIG. 22 is a partial exploded structural schematic view of the skin care assembly according to another embodiment of the present disclosure.

In some embodiments, the heat dissipation member 300 includes a second cover 300a and a heat dissipation body 300b. The first guidance portion 350, the second guide portion 360, and the second fastener 310 are disposed on the second cover 300a. The second cover 300a is disposed between the heat dissipation body 300b and the first cover 500. The second cover 310 is fixed on the heat dissipation body 300b. The first cover 500 is fixed on the second cover 300a.

Optionally, thermal coupling surface 340 is disposed on the heat dissipation body 300b. The third side surface 320 and the fourth side surface 330 connected with the two sides of thermal coupling surface 340 are disposed on the second cover 300a. The second cover 300a is disposed between the heat dissipation body 300b and the first cover 500. The second cover 310 is fixed on the heat dissipation body 300b. The first fastener 520 is fastened into the first fastener 310, such that the first cover 500 is fixed on the second cover 300a. In this way, the following functions may be achieved, that is, the second cover 300a is configured to connect to the first cover 500 and protect the heat dissipation body 300b, and the heat dissipation body 300b is configured to connect to the refrigeration member 200 and dissipate heat, such that it is more convenient to install and align when the skin care assembly 10 is assembled with the first cover 500 and the heat dissipation member 300, and it is also convenient to replace accessories.

Optionally, as shown in FIGS. 18-20, the skin care assembly 10 further includes a lead 230 and a recess 380. The lead 230 is connected and fixed to a side of the refrigeration member 200. The recess 380 is disposed on an inner side of the third side surface 320 or the fourth side surface 330 of the second cover 300a. Optionally, the number of the leads 230 is at least one. The number of the recesses 380 is the same as that of the leads 230. Further, the lead 230 is received in the recess 380 during an installation process of the skin care assembly 10, such that the refrigeration member 200 is connected and fixed to the heat dissipation member 300.

Optionally, as shown in FIG. 19, the second cover 300a further defines a restriction hole 300c. The restriction hole 300c is located on and in communication with the opening 510. During an installation process of the skin care assembly 10, the heat generating side 220 of the refrigeration member 200 is partially disposed in the restriction hole 300c, such that the refrigeration member 200 is fixed on the second cover 300a. Optionally, a distance between an edge of the refrigeration member 200 and an edge of the restriction hole 300c is in a range from 0.5mm to 1mm. For example, the distance between an edge of the refrigeration member 200 and an edge of the restriction hole 300c may be 0.5mm, 0.75mm, or 1mm, such that a supporting area of the edge of the restriction hole 300c on the second cover 300a is enough to fix the refrigeration member 200.

Optionally, the recess 380 is disposed along an edge of the restriction hole 300c, such that the lead 230 fixed on the refrigeration member 200 may be inserted into the restriction hole 300c and received in the recess 380. Optionally, a ratio of a pore area of the restriction hole 300c to an area of the opening 510 is in a range from 0.8 to 1.2. For example, the pore area of the restriction hole 300c to the area of the opening 510 is 0.8, 1.0 or 1.2, such that the opening 510 of the first cover 500 is completely corresponding to the restriction hole 300c of the second cover 300a in the axial direction, and thus it is more convenient to install and align when the skin care assembly 10 is assembled with the first cover 500 and the heat dissipation member 300, and it is also convenient to replace accessories.

In the skin care assembly 10 provided by some embodiments of the present disclosure, the electrode heads 400 is fixed on the cold compress member 100, and the cold compress member 100 and the refrigeration member 200 are fixed on the heat dissipation member 300 via the first cover 500, such that the heat generated by the skin may be conducted rapidly to the heat dissipation member 300, thereby dissipating the heat from the skin care assembly. In addition, the cold compress member 100 and the refrigeration member 200 are fixed in the heat dissipation member 300 with the simple structure, thereby saving the internal space of the skin care component 10, and reducing the production difficulty. Further, the first fastener 520 is disposed on the first cover 500, and the second fastener 310 is disposed on the heat dissipation member 300, such that it is more convenient to install and align when the skin care assembly 10 is assembled with the first cover 500 and the heat dissipation member 300, and it is also convenient to replace accessories.

The skin care assembly may also be provided in some embodiments of the present disclosure. The skin care assembly includes the above-mentioned skin care assembly 10 and a shell. Optionally, the skin care assembly 10 is disposed in the shell. The shell is configured to separate an internal structure of the skin care assembly 10 from an external part, which is convenient for use.

Optionally, the skin care assembly may be a beauty apparatus or a beauty device. The cold compress member 100 may be sapphire material. When the beauty apparatus is in operation, the electrode heads 400 of the skin care assembly 10 may be discharged on the skin, such that the temperature of the skin increase. By thermally coupled connection and fixing the first cover 500, the heat generated by the skin may be dissipated from the skin care assembly via the cooperation between the cold compress member 100, the refrigeration member 200, and the heat dissipation member 300. The first fastener 520 of the first cover 500 is fastened into or out the second fastener 310 of the heat dissipation member 300, thereby installing and aligning the skin care assembly or replacing the accessories. The skin care assembly is assembled with the device body via the shell, such that the skin care assembly may be applied to various beauty devices.

In some embodiments of the present disclosure, the following technical effects of the skin care assembly 10 may be achieved.

In some embodiments of the present disclosure, the skin care assembly 10 has the technical effect of facilitating installation and reducing the production difficulty. The one or more electrode heads 400 are fixed to the cold compress member 100, which reduces redundant structures and makes the design more concise. Without the interference of the redundant structures, the skin are also more comfortable to the touch. In addition, the cold compress member has the good conduction performance, which is configured to the heat generated by the one or more electrode heads 400 stimulating the epidermis, thereby cooling the skin epidermis, and achieving the cold compress and cooling function. In this way, it is possible to reduce the situation that the skin is burned due to excessive temperature, thereby reducing the discomfort during use and improving the operating comfort of the operation head. At the same time, the cold compress member 100, the refrigeration member 200, and the heat dissipation member 300 are thermally coupled with each other, and fixed via the first cover 500, such that the heat generated by the skin may be dissipated out rapidly. Installation structures of the cold compress member 100 and the refrigeration member 200, and the heat dissipation member 300 are simple, thereby saving the internal space of the skin care component 10 and reducing the production difficulty. A suitable temperature environment is provided by the cooperation of cold compress member 100 and the refrigeration member 200, and the heat dissipation member 300, which further improves the beauty effect. The first fastener 520 is disposed on the first cover 500, and the second fastener 310 is disposed on the heat dissipation member 300, such that it is more convenient to install and align when the skin care assembly 10 is assembled with the first cover 500 and the heat dissipation member 300, and it is also convenient to replace accessories. The skin care assembly may also be provided in some embodiments of the present disclosure. The skin care assembly includes the above-mentioned skin care assembly and the shell. The skin care assembly is disposed in the shell.

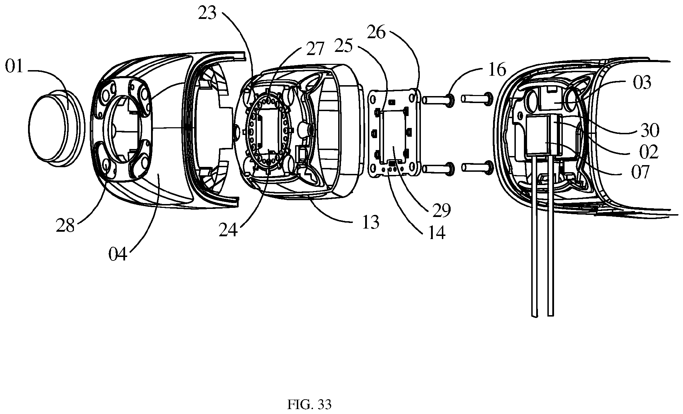

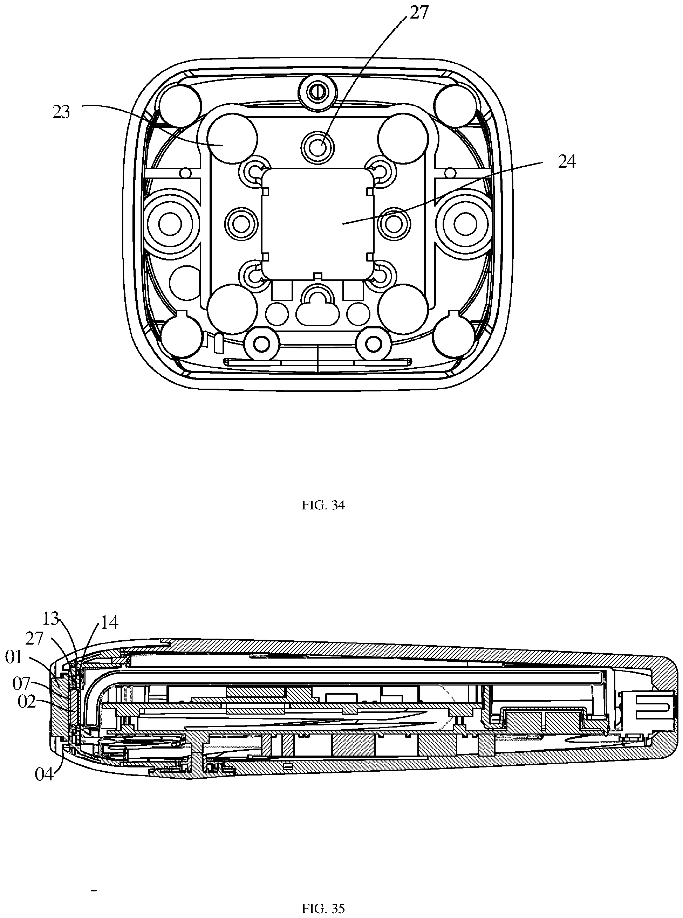

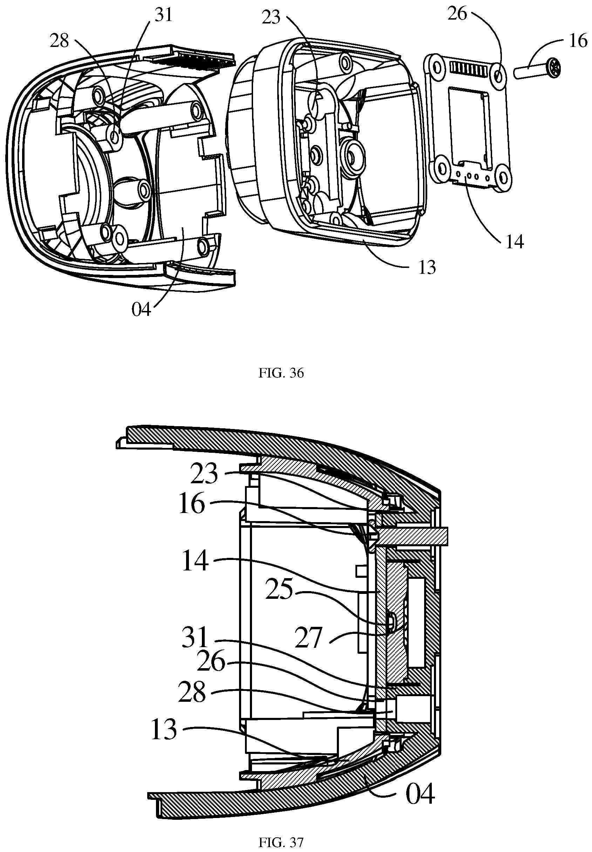

FIG. 33 is an exploded structural schematic view of the skin care assembly according to another embodiment of the present disclosure. FIG. 34 is a structural schematic view of a light guide plate close to a side of a phototherapeutic light board of the skin care assembly according to an embodiment of the present disclosure, showing a side of the light guide plate close to a phototherapeutic light board. FIG. 35 is a cross-sectional schematic view of the skin care assembly according to another embodiment of the present disclosure. FIG. 36 is an exploded schematic view of the first cover, the light guide plate, the phototherapeutic light board, and a first bolt of the skin care assembly according to an embodiment of the present disclosure. FIG. 37 is a cross-sectional schematic view of the first cover, the light guide plate, the phototherapeutic light board, and the first bolt of the skin care assembly in an assembled state according to an embodiment of the present disclosure.

As shown in FIGS. 33-35, the skin care assembly further includes a phototherapeutic light board 14. One or more phototherapeutic lights 25 are arranged on a side of the phototherapeutic light board 14 close to a first cover 04. A first through hole 29 is defined on a center of the phototherapeutic light board 14. The one or more phototherapeutic lights 25 are arranged around the first through hole 29. Further, the one or more phototherapeutic lights 25 may be just covered by a projection of a cold compress member 01 substantially perpendicular to a direction of a contact surface 01a. A refrigeration member 02 and a cold-guiding member 07 may pass through the first through hole 29 and contact with the cold compress member 01. The phototherapeutic light board 14 is arranged opposite to a heat dissipation member 03. A side of the phototherapeutic light board 14 which there is no the phototherapeutic light 25 located on is close to and thermally coupled with the heat dissipation member 03. In addition, the first through hole 29 is configured to limit a position of a first restriction portion 30 protruding from the heat dissipation member 03, such that the phototherapeutic light board 14 and the heat dissipation member 03 are relatively fixed. Further, the first restriction portion 30 is thermally coupled with the refrigeration member 02.

Optionally, a light guide plate 13 may be disposed between the phototherapeutic light board 14 and the first cover 04. A second through hole 24 is defined on a center of the light guide plate 13 and disposed opposite to the first through hole 29. One or more light guide holes 27 are arranged around the second through hole 24. The one or more light guide holes 27 are arranged corresponding to the one or more phototherapeutic lights 25. Further, the one or more light guide holes 27 may be just covered by the projection of the cold compress member 01 substantially perpendicular to the direction of the contact surface 01a. The one or more light guide holes 27 are covered with light-transmitting materials, and the light emitted by the one or more phototherapeutic lights 25 may be reflected on the cold compress member 01 via the one or more light guide holes 27. The refrigeration member 02 and the cold-guiding member 07 may pass through the second through hole 24 and contact with the cold compress member 01, and the second through hole 24 is configured to limit a position of the refrigeration member 02. One or more a first fixing holes 26 are defined on the phototherapeutic light board 14. Each of the one or more a first fixing holes 26 is disposed opposite to a third fixing hole 28 of the first cover 04, thereby fixing the phototherapeutic light board 14 relatively to the first cover 04.

As shown in FIG. 36 and FIG. 37, the phototherapeutic light board 14 is relatively fixed to the first cover 04 and the light guide plate 13 by means of snapping, bolts, tenon joints or threaded bolts. In some embodiments, a connection method is threaded bolts. Inner walls of the first fixing holes 26 and the third fixing holes 28 are also arranged with threads corresponding to the first bolts 16. One or more first bolts 16 may pass through the first fixing holes 26 and the third fixing holes 28 to relatively fix the phototherapeutic light board 14 to the first cover 04.

Optionally, one or more second fixing holes 23 are defined on the light guide plate 13. The number of the second fixing holes 23 is equal to that of the first fixing holes 26/the third fixing holes 28/the first bolts 16. A first restriction member 31 is arranged on a side of the first cover 04 close to the light guide plate 13. A shape and a size of the first restriction member 31 are substantially the same as a shape and an inner diameter of the second fixing hole 23. That is, a position of the first restriction member 31 may be limited by the second fixing hole 23. The third fixing hole 28 is inserted into the first restriction member 31, such that the phototherapeutic light board 14, the light guide plate 13, and the first cover 04 may be relatively fixed by the first bolts 16 in the sequence.

Optionally, the one or more phototherapeutic lights 25 may be LED light-emitting lamp beads. During the one or more phototherapeutic lights 25 are in operation, the emitting light appears as red light when the emitting light is emitted to the cold compress member 01 via the one or more light guide holes 27. At this time, the cold compress member 01 appears as a red luminous body, which may produce a phototherapy effect on the skin. Meanwhile, the one or more phototherapeutic lights 25 may also have other functions, such as removing hair.

Optionally, the light-transmitting materials covered on the one or more light guide holes 27 may be mostly organic or inorganic materials with good light-transmitting properties, such as various optical plastics, silicate glass, and the like.

Optionally, in some embodiments, there is no light guide plate 13 disposed between the phototherapeutic light board 14 and the first cover 04. The light emitted by the one or more phototherapeutic lights 25 may be directly mapped to the cold compress member 01.

Optionally, the skin care assembly 1 may further include a second cover 08, a first installation plate, a second installation plate, a shell 11, a button 12, a charging port 10, a thermally conductive housing 09, and the like. In some embodiments of the present disclosure, the sapphire may be either artificial or natural.

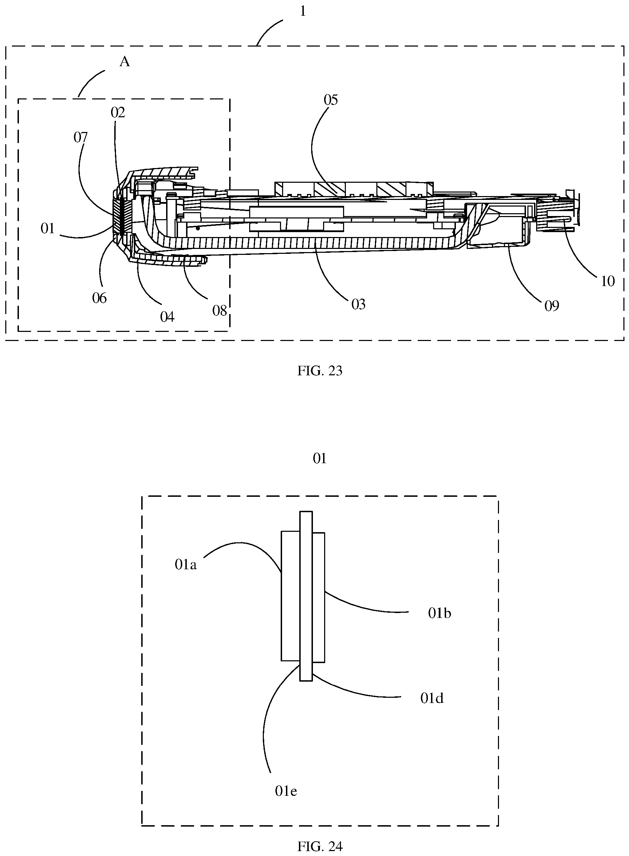

As shown in FIG. 23, FIG. 23 is a cross-sectional structural schematic view of the skin care assembly according to an embodiment of the present disclosure. The skin care assembly 1 includes: a cold compress member 01 having a contact surface 01a which is configured to contact the skin, and a conduction surface 01b, a refrigeration member 02, a heat dissipation member 03, a first cover 04, a pulse generating circuit 05, and an electrode 06. The cooling side of the refrigeration member 02 is thermally coupled with the conduction surface 01b of the cold compress member 01, thereby cooling the cold compress member 01. A heat generating side of the refrigeration member 02 is thermally coupled with the heat dissipation member 03 to dissipate heat generated by the refrigeration member 02. The first cover 04 is configure to relatively fix the cold compress member 01 to the heat dissipation member 03, and the cold compress member 01 and the heat dissipation member 03 are partially wrapped by the first cover 04. The pulse generating circuit 05 is disposed on a side of the heat dissipation member 03 opposite to the refrigeration member 02, so as to generate electric/magnetic pulses. The electrode 06 is relatively fixed to the first cover 04 and protrudes from the first cover 04, and is connected to the pulse generating circuit 05. When the electrode 06 contacts the skin, the pulse generating circuit 05 generates the electric/magnetic pulses to care the skin.

In the skin care assembly 1 provided by some embodiments of the present disclosure, the cold compress member 01 and the electrode 06 are fixed on the first cover 04, such that the cold compress member 01 and the electrode 06 are better fixed, thereby increasing structural stability of the cold compress member 01 and the electrode 06. At the same time, the refrigeration member 02 and the pulse generating circuit 05 are arranged on the heat dissipation member 03, and the pulse generating circuit 05 is disposed on a side of the heat dissipation member 03 opposite to the refrigeration member, which improves the heat dissipation efficiency of the skin care assembly 1 and reduces the interference of the heat generated by the refrigeration member 02 and the pulse generating circuit 05 to the cold compress member 01. The electrode 06 is fixed on the first cover 04, such that it is not necessary to process and install structures of the electrode 06 on elements such as the cold compress member 01, which reduces the processing cost accordingly.

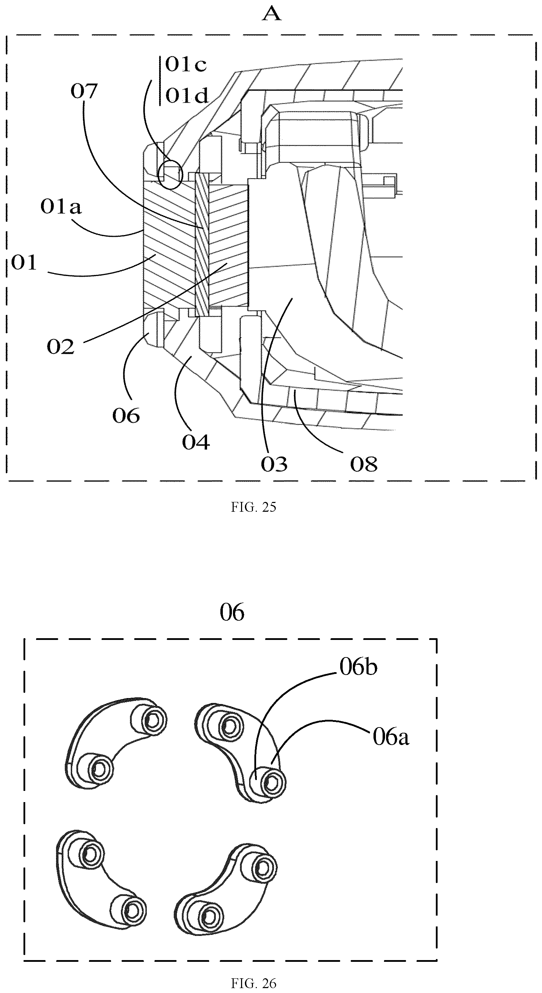

Specifically, as shown in FIG. 24 and FIG. 25, FIG. 24 is a side schematic view of a cold compress member as shown in FIG. 23, and FIG. 25 is a partial enlarged schematic view of the skin care assembly as shown in FIG. 23 at a position A. The first cover 04 includes two side surfaces, namely, an outside and an inner side. The outside is exposed to the air and is configured to fix the electrode 06 and the cold compress member 01. The inner side is configured to wrap the refrigeration member 02, and partially wrap the heat dissipation member 03 and the cold compress member 01. Four first installation holes 04b are defined on the inner side of the first cover 04, so as to fix the first cover 04. The heat dissipation member 03 has a first end, and four second installation holes 04b are defined on the first end of the heat dissipation member 03. After the electrode 06 and the cold compress member 01 are installed on the first cover 04, the first installation holes 04b defined on the first cover 04 align g to the four second installation holes defined on the heat dissipation member 03 so as to install and fix the first cover 04 and the heat dissipation member 03 by screws. After installation, the first end of the heat dissipation member 03 is wrapped in the inner side of the first cover 04 by the first cover 04. The heat generating side of the refrigeration member 02 is connected to and thermally coupled with the first end of the heat dissipation member 03, and the heat generating side and the first end are wrapped by the first cover 04. The cooling side of the refrigeration member 02 is thermally coupled with the conduction surface 01b of the cold compress member 01, and the cooling side and the conduction surface 01b are wrapped by the first cover 04. The conduct surface 01a of the cold compress member 01 and the electrode 06 are disposed on the outside of the first cover 04, such that it is possible to ensure that the skin care assembly 1 may perform electromagnetic pulse therapy on the skin through the electrode 06 during operation. At the same time, the skin care assembly 1 may also cool and protect the skin through the cold compress member 01, thereby improving the skin care effect of the skin care assembly 1. An accommodating cavity is defined between the first end and a second end of the heat dissipation member 03. The above-mentioned second end of the heat dissipation member 03 is an end opposite to the heat generating side of the refrigeration member 02 and connected to the first end of the heat dissipation member 03. A support frame is disposed in the accommodating cavity. The pulse generating circuit 05 is fixed on the support frame of the heat dissipation member 03. When the skin care assembly 1 is in operation, the heat generated by the cooling 02 and the pulse generating circuit 05 is directly conducted to the heat dissipation member 03, such that the heat is dissipated out by the heat dissipation member 03 with good heat dissipation performance.

Optionally, the number of the electrodes 06 is at least two, and the at least two electrodes 06 are arranged on a peripheral side of the cold compress member 01 and spaced apart from each other. The cold compress member 01 is relatively fixed to the heat dissipation member 03 by the first cover 04 via the electrode 06. An electromagnetic pulse is the radio frequency and/or the micro-current.

Specifically, as discussed above, the first cover 04 includes the two side surfaces, namely, the outside and the inner side. In addition, the outside is in communication with the inner side via the opening. An installation table is arranged on the outside of the first cover 04. The cold compress member 01 is pressed and fixed on the installation table of the outside of the first cover 04 via the electrode 06. At the same time, a first accommodating cavity is defined on the inner side of the first cover 04. In the skin care assembly 01, some components of the heat dissipation member 03 are wrapped by the first cover 04 or partially wrapped in the first accommodating cavity of the first cover 04. The conduction surface 01b passes through an opening defined between the outside and the inner side, and abuts against a side of the heat dissipation member 03. The number of the electrodes 06 is four. The four electrodes 06 are evenly arranged on the peripheral side of the cold compress member 01. The electrodes 06 are fixed on the installation table of the outside of the first cover 04, at the same time, the cold compress member 01 is pressed and fixed on the installation table of the outside of the first cover 04 via an edge of each of the electrodes 06. The cold compress member 01 is fixed via the plurality of electrode 06. A plurality of electrodes 06 are configured to fix the cold compress member 01, such that it is possible to improve the installation stability of the cold compress member 01 when the cold compress member 01 is fixed on the installation table of the outside of the first cover 04, the assembly of which is simple. When the cold compress member 01 dissipates heat to the skin, the cold compress member 01 may also dissipate heat to the electrode 06 at the same time, such that the temperature of the parts in contact with the skin is more uniform, thereby improving the comfort.

Optionally, a first stepped surface 06a is disposed on a side of the electrode 06 facing towards the cold compress member 01. A second stepped surface 01c is disposed on a side of the cold compress member 01. The second stepped surface 01c is located between the first stepped surface 06a and the heat dissipation member 03. The first stepped surface 06a abuts against the second stepped surface 01c.

Specifically, as shown in FIGS. 24-27, in some embodiments, the electrode 06 is arranged in a crescent shape and has two special ends, one end is a functional end and configured to care the human skin by using electric/magnetic pulses during operation, and the other end is a fixing end and is connected to the first cover 04 to fix the electrode 06 on the installation table of the outside of the first cover 04. The first stepped surface 06a is disposed between the functional end and the fixing end. During installation, the first stepped surface 06a faces towards the installation table of the outside of the first cover 04, at the same time, a fourth stepped surface 01e is disposed between the contact surface 01a and the conduction surface 01b of the cold compress member 01. During installation, the cold compress member 01 is installed on the installation table of the first table, the second stepped surface 01c faces a direction away from the inner side of the first cover 04, and then the electrodes 06 are installed on the installation table of the first cover 04 one by one around the cold compress member 01. After installation, when the first stepped surface 06a of the electrode 06 just presses on the fourth stepped surface 01e of the cold compress member 01, such that the cold compress member 01 is pressed and fixed on the installation table of the first cover 04. After the installation is completed, the second stepped surface 01c of the cold compress member 01 is located between the first stepped surface 06a of the electrode 06 and the installation table of the first cover 04, that is, the second stepped surface 01c is located between the first stepped surface 06a and the heat dissipation member 03.

Optionally, an opening is defined on the first cover 04. A fifth stepped surface is disposed on the opening. A sixth stepped surface 01d is disposed on the side of the cold compress member 01. The sixth stepped surface 01d is opposite to or facing away from the fourth stepped surface 01e. The sixth stepped surface 01d is located between the fifth stepped surface and the electrode 06. The fifth stepped surface abuts against the sixth stepped surface 01d.

Specifically, the opening is defined on the first cover 04. The inner side of the first cover 04 is in communication with the installation table of the outside via the opening. The fifth stepped surface is disposed around the installation table of the outside of the first cover 04 close to the opening. At the same time, the sixth stepped surface 01d is disposed between the second stepped surface 01c of the cold compress member 01 and the conduction surface 01b. During installation, the fifth stepped surface abuts against the sixth stepped surface 01d, such that the cold compress member 01 is fixed entirely on the installation table of the outside of the first cover 04. At the same time, the conduction surface 01b of the cold compress member 01 may pass through the opening and relatively fix the heat dissipation member 03.

Optionally, the electrode 06 is fixed on the first cover 04. The cold compress member 01 is pressed against the opening of the first cover by the electrode 06. The cold compress member 01 abuts against the electrode 06 and the first cover 04 in two directions opposite to each other or facing away from each other. The cold compress member 01 is clamped between the electrode 06 and the first cover 04. In this way, it is not necessary to design an additional installation structure on the cold compress member 01, which reduces its processing cost, and this method is easy to install. At the same time, the force around the cold compress member 01 is uniform, such that the structure is stable, and it is not easy to be damaged.

Optionally, the electrode 06 includes at least one installation post 06b. The first cover 04 defines at least one third installation hole. The installation post 06b is installed in the installation hole to fix the electrode 06 on the first cover 04. A height of the electrode 06 protruding from the first cover 04 is in a range from 0mm to 20mm. In this way, when the user uses the skin care assembly, the electrode 06 and the cold compress member 01 may stick to the skin, and there is no discomfort.

Specifically, the fixing end of the electrode 06 is arranged with two installation posts 06b. Two third installation holes are arranged corresponding to the installation posts 06b of the electrode 06 on the installation table of the first cover 04. The installation table of the first cover 04 is totally arranged with four pairs of third installation holes corresponding to the installation posts 06b of the electrodes 06. The four pairs of third installation holes are arranged around the opening of the first cover 04. In addition, the first stepped surface 06a the second stepped surface 01c. It is possible to ensure that the first stepped surface 06a defined on the electrode 06 may fit with the second stepped surface 01c defined on the cold compress member 01 during installation, and the electrode 06 may be pressed to fix the cold compress member 01 on the first cover 04. After the cold compress member 01 and the electrode 06 are fixed the first cover 04, the height of the functional end of the electrode 06 protruding from the first cover 04 is in the range from 0mm to 20mm.

Optionally, in some embodiments, the skin care assembly 1 further includes a cold-guiding member 07. The cold-guiding member 07 is disposed between the cold compress member 01 and the refrigeration member 02, and is configured to conduct heat between the cold compress member 01 and the refrigeration member 02. The cold-guiding member 07 abuts against the cold compress member 01.

Specifically, the cold-guiding member 07 is disposed between the conduction surface 01b of the cold compress member 01 and the cooling side of the refrigeration member 02. The cold-guiding member 07 has good thermal conductivity, such that the refrigeration member 02 efficiently cools the cold compress member 01 through the cold-guiding member 07.

Optionally, in some embodiments, the skin care assembly 1 further includes the first cover 04, which is partially disposed between the first cover 04 and the heat dissipation member 03. The second cover 08 is fixed on the heat dissipation member 03, and the first cover 04 is fixed on the second cover 08. The cold-guiding member 07 and the refrigeration member 02 are pressed against the heat dissipation member 03 in the first direction via the cold compress member 01.

Specifically, the second cover 08 is sleeved on the inner side of the first cover 04 and is partially disposed between the first cover 04 and the heat dissipation member 03, thereby limiting a movement of the heat dissipation member 03 in a first direction. The first direction mentioned here is a direction substantially perpendicular to the first cover 04 and the second cover 08 at the same time. The second cover 08 defines four first positioning holes corresponding to the first installation holes and the second installation holes. Four positioning posts 04a are arranged on the inner side of the first cover 04. In addition, the first installation holes 04b defined on the first cover 04 are arranged in the four positioning posts 04a. During installation, the four positioning posts defined on the first cover 04 pass through the first positioning holes defined on the second cover 08 and abuts against on the second positioning holes defined on the heat dissipation member 03, which are fixed via the screws, such that the second cover 08 is fixed between the first cover 04 and the heat dissipation member 03. The cold compress member 01 fixed on the first cover 04 passes through the first cover 04 and the cold-guiding member 07 and presses against the heat dissipation member 03 in the direction substantially perpendicular to the first cover 04 and the second cover 08 at the same time, namely, the first direction.

Optionally, the second cover 08 defines a restriction hole. The restriction hole is located on and in communication with the opening of the first cover 04. The refrigeration member 02 is partially disposed in the restriction hole, and a distance between an edge of the refrigeration member 02 and an edge of the restriction hole is in a range from 0.5mm to 1mm. A recess is defined on an area of the heat dissipation member 03 corresponding to the refrigeration member 02. The recess is arranged corresponding to the restriction hole. One part of the refrigeration member 02 is embedded in the recess, and the other part of the refrigeration member 02 is disposed in the restriction hole. In addition, a ratio of an area of the restriction hole to an area of the opening is in a range from 0.8 to 1.2.

Specifically, the second cover 08 defines a restriction hole corresponding to the opening of the first cover 04 in the first direction. After the skin care assembly 1 is installed, the restriction hole and the opening of the first cover 04 are located in the first direction and in communication with each other, such that the refrigeration member 02 may be thermally coupled with the cold compress member 01, thereby cooling the cold compress member 01. The first end of the heat dissipation member 03 defines a recess corresponding to the refrigeration member 02, and a part of the refrigeration member 02 is embedded in the recess. The refrigeration member 02 passes through the restriction hole defined on the second cover 08 and is connected to the cold-guiding member 07, thereby cooling the cold compress member 01.

Optionally, the above-mentioned skin care assembly further includes thermally conductive housing 09. Thermally conductive housing 09 is installed the second end of the heat dissipation member 03 and configured to improve the heat dissipation efficiency.

Specifically, an installation post is disposed on the second end of the heat dissipation member 03. The installation post defines a third positioning hole. An end of thermally conductive housing 09 defines a fourth positioning hole. After the third positioning hole fits with the fourth positioning hole during installation, thermally conductive housing 09 is fixed on a side wall of the second end of the heat dissipation member 03 via screws. A side wall of the assembled thermally conductive housing 09 fits with the second end of the heat dissipation member 03, such that the heat generated by the heat dissipation member 03 may be efficiently conducted to thermally conductive housing, thereby ensuring the heat dissipation efficiency and working stability of the skin care assembly 1.

Optionally, the above-mentioned skin care assembly 1 further includes a charging port 10. The charging port 10 is installed on the pulse generating circuit 05 and is disposed on a side of the second end of the heat dissipation member 03. The charging port 10 is configured to charge the skin care assembly 1.

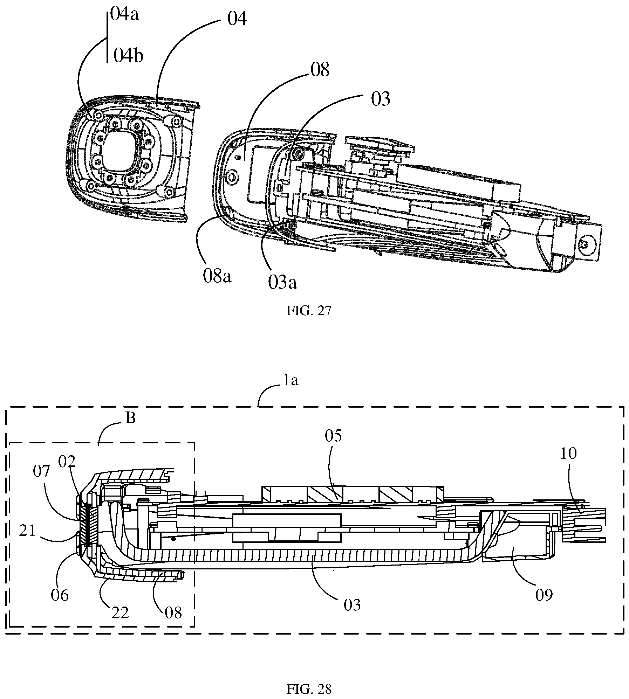

As shown in FIG. 28, another embodiment may be further provided. FIG. 28 is a cross-sectional structural schematic view of the skin care assembly according to another embodiment of the present disclosure. The skin care assembly 1 includes: a cold compress member 01 having a contact surface 01a which is configured to contact the skin, and a conduction surface 01b, a refrigeration member 02, a heat dissipation member 03, a first cover 04, a pulse generating circuit 05, and an electrode 06. The cooling side of the refrigeration member 02 is thermally coupled with the conduction surface 01b of the cold compress member 01, thereby cooling the cold compress member 01. A heat generating side of the refrigeration member 02 is thermally coupled with the heat dissipation member 03 to dissipate heat generated by the refrigeration member 2. The first cover 04 is configure to relatively fix the cold compress member 01 to the heat dissipation member 03, and the cold compress member 01 and the heat dissipation member 03 are partially wrapped by the first cover 04. The pulse generating circuit 05 is disposed on a side of the heat dissipation member 03 opposite to the refrigeration member, so as to generate electric/magnetic pulses. The electrode 06 is relatively fixed to the first cover 04 and protrudes from the first cover 04, and is connected to the pulse generating circuit. When the electrode 06 contacts the skin, the pulse generating circuit 05 generates the electric/magnetic pulses to care the skin.

Optionally, an installation structure of the electrodes 06, the first cover 04, and the cold compress 01 may also be set as follows. The number of the electrodes 06 is at least two, and at least two electrodes 06 are arranged on the first cover 04 at intervals. The electrodes 06 are fixed on the first cover 04, and the first cover 04 defines an opening. A seventh stepped surface 22a is disposed on the opening. An eighth stepped surface 21c is disposed on a side of the cold compress member 01 close to the opening. The seventh stepped surface 22a abuts against the eighth stepped surface 21c. The eighth stepped surface is located between the first cover 04 and the refrigeration member 02.

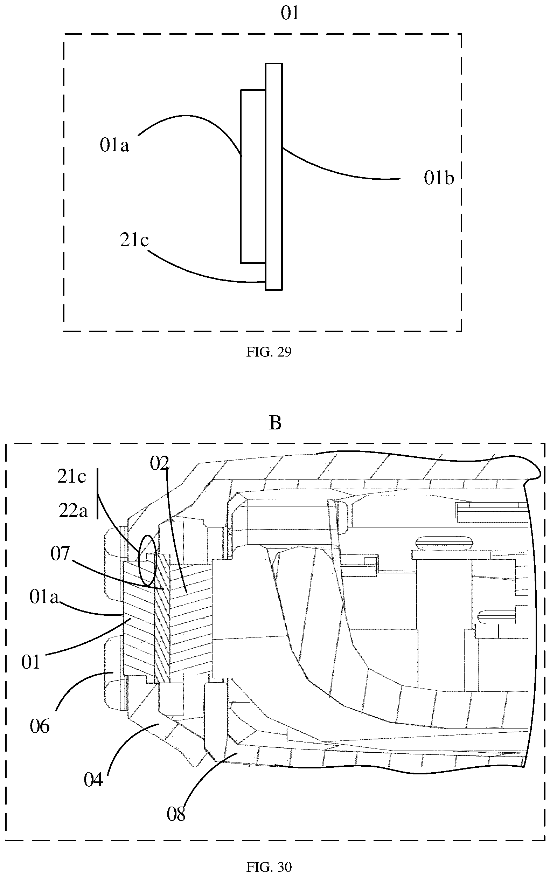

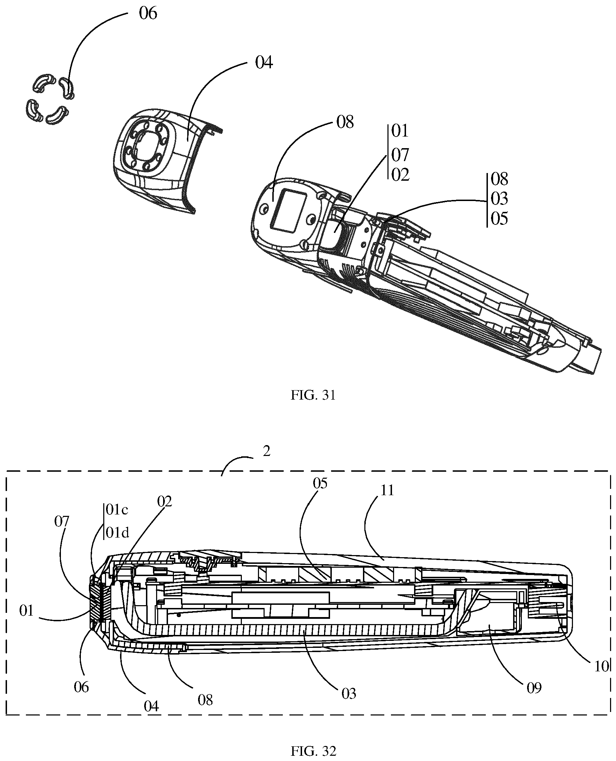

Specifically, as shown in FIGS. 29-31, FIG. 29 is a side schematic view of the cold compress member as shown in FIG. 28, FIG. 30 is a partial enlarged schematic view of the skin care assembly as shown in FIG. 28 at a position B, and FIG. 31 is an exploded structural schematic view of the skin care assembly according to another embodiment of the present disclosure. The first cover 04 includes two side surfaces, namely, an outside and an inner side. Different from the first embodiment, in the second embodiment, the eighth stepped surface 21c is disposed on the cold compress member 01. The seventh stepped surface 22a is disposed on the inner side of the first cover 04. The installation table is arranged on the outside of the first cover 04. The electrode 06 is installed on the installation table of the outside of the first cover 04 and is configured to care the skin. The refrigeration member 02, the cold-guiding member 07, and the cold compress member 01 sequentially abuts against the first end of the heat dissipation member 03. Further, the seven stepped surface 22a of the inner side of the first cover 04 abuts against and fits with the eighth stepped surface 21c defined on the cold compress member 01, such that the refrigeration member 02, the cold-guiding member 07, and the cold compress member 01 are sequentially fixed between the first cover 04 and the heat dissipation member 03. In addition, the contact surface 01a of the cold compress member 01 passes through the opening of the first cover 04 and is located on the outside of the first cover 04, which is configured to help the electrode 06 to care the skin.

As described above, in the second embodiment, except for the above-mentioned differences, other features are the same as those of the first embodiment, and will not be repeated here.

In the skin care assembly 1 provided by some embodiments of the present disclosure, the electrode 06 is installed on the installation table of the outside of the first cover 04. The cold compress member 01 is pressed and fixed between the first cover 04 and the side of the heat dissipation member 03 via the first cover 04, such that the cold compress member 01 and the electrode 06 are better fixed, thereby increasing structural stability of the cold compress member 01 and the electrode 06. At the same time, the refrigeration member 02 and the pulse generating circuit 05 are arranged on the heat dissipation member 03, and the pulse generating circuit 05 is disposed on a side of the heat dissipation member 03 opposite to the refrigeration member, which improves the heat dissipation efficiency of the skin care assembly 1 and reduces the interference of the heat generated by the refrigeration member 02 and the pulse generating circuit 05 to the cold compress member 01. The electrode 06 is fixed on the first cover 04, such that it is not necessary to process and install structures of the electrode 06 on elements such as the cold compress member 01, which reduces the processing cost accordingly.

Different from the related art, in the skin care assembly provided in some embodiments of the present disclosure, the electrode 06 and the cold compress member 01 are two major functional members, and are installed on the first cover 04 by the method of fixing to each other, such that it is possible to ensure that the electrode 06 and the cold compress member 01 may be installed firmly, and also ensure that the functional end of the electrode 06 and the contact surface 01a of the cold compress member 01 may simultaneously contact the skin during operation. In this way, when the skin is cared, the skin care assembly 1 may rapidly and evenly cool the skin via the electromagnetic pulses, thereby improving the effect of skin care. Furthermore, in some embodiments of the present disclosure, the position of the heat dissipation member 03 may be limited by the second cover 08, such that the heat dissipation member 03 may always keep a certain distance from the cold compress member 01. At the same time, the restriction hole is defined on the second cover 08. Therefore, with the above-mentioned special structures, it is possible to effectively ensure that the heat generated by main heating members is transferred to the heat dissipation member 03, thereby ensuring that the refrigeration member 02 may efficiently cool the cold compress member 01.

A contact-type skin care device is provided in some embodiments of the present disclosure. As shown in FIG. 32, FIG. 32 is a cross-sectional structural schematic view of a contact-type skin care device according to an embodiment of the present disclosure. The contact-type skin care device 2 includes all the technical features of the above-mentioned skin care assembly. The contact-type skin care device 2 further includes a housing 11, and at least part of the skin care assembly is disposed in the housing 11. A beauty device is also provided in some embodiments of the present disclosure. The beauty device includes a heating member, a cold compress member, a refrigeration member, and a heat dissipation member. The heating member heats the skin electrically, magnetically, or electromagnetically. The cold compress member includes a cooling surface and a conduction surface. The cooling surface is configured to cool at least part of the skin or adjacent tissue of the skin. The cooling side of the refrigeration member is thermally coupled with the conduction surface of the cold compress member, so as to obtain the heat of the cold compress member. The heat dissipation member is thermally coupled with the heat generating side of the refrigeration member, so as to dissipate the heat out. The skin or skin surface close to the skin is cooled by the cold compress member. An action depth range of electricity, magnetism or electromagnetism on the skin is larger than that of electricity, magnetism or electromagnetism on the skin surface. In addition, at the same time, an area of a cooling surface is greater than an action area of electricity, magnetism or electromagnetism on the skin. A working power of the heating member meets the following condition: the temperature of heating the skin tissue below the surface is in a range from 45 °C to 80 °C. A working power of the cold compress member meets the following condition: the surface temperature is reduced to a range from 15 °C to 45 °C.

In the beauty device, the skin is heated by the heating member, the skin is cooled by the cold compress member, the cold compress member is cooled by the refrigeration member, and the heat generated by the refrigeration member is dissipated by the heat dissipation member. The skin or adjacent tissue of the skin is cooled by the cold compress member,

Optionally, the heating member heats the skin via a contact type or non-contact type. It is not necessary for the cold compress member to cool the skin, as long as the temperature of the skin may be reduced. The action depth range of electricity, magnetism or electromagnetism on the skin is larger than that of electricity, magnetism or electromagnetism on the skin surface, such that different temperature and stimulation effects at different depths and levels of the skin may be achieved. For example, the temperature of heating the skin tissue below the surface is in the range from 15 °C to 80 °C, and then the epidermis temperature rises with the temperature of deep layer increasing. Since there may be one or more groups of the electrodes, there may be only some electrodes in operation at the same time. As long as it is satisfied that the area of the cooling surface is larger than the action area of the electricity, magnetism, or electromagnetism on the skin at the same time. In this way, the cooling surface may fully and evenly cool the skin, thereby reducing unpleasant sensations such as a tingling sensation caused by excessively high temperature or uneven temperature. The heating member and the cold compress member work together to make the temperature of the skin tissue and the epidermis below the surface reach a balance, thereby achieving a better beauty effect. For example, the working power of the heating member meets the following condition: the temperature of heating the skin tissue below the surface is in the range from 45 °C to 80 °C. The working power of the cold compress member meets the following condition: the surface temperature is reduced to the range from 15 °C to 45 °C. Therefore, a temperature balance with a good beauty effect may be achieved.

Optionally, the contact surface of the cold compress member is a contact surface contacting at least part of the skin or the adjacent tissue of the skin. The heating member is an electrode group. The electrode group has an end surface contacting the skin. The total area of the end surface is smaller than that of the contact surface.

Optionally, the working power of the heating member meets the following condition: the temperature of heating the skin tissue below the surface is in the range from 50 °C to 65 °C;

The working power of the cold compress member meets the following condition: the surface temperature is reduced to the range from 15 °C to 45 °C.

The above-mentioned technical solutions and embodiments may be combined arbitrarily without contradiction.

Optionally, in some embodiments of the present disclosure, the above-mentioned beauty device, the skin care assembly, or the like, includes the refrigeration member, and cannot include the cold compress member. At this time, the cooling side of the refrigeration member may directly contact with the skin.

Optionally, on the basis that the cold compress member is not required, the cooling side of the refrigeration member may be covered with some medium layers, such as insulating layers.

The above is only an implementation of the present disclosure, not to limit the scope of the present disclosure. Any equivalent structure or equivalent process transformation made by using the content of the specification and the accompanying drawings of the present disclosure, or directly or indirectly applied in other related technical fields, are included in the scope of the present disclosure in the same way.

## Claims

1. A skin care assembly comprising: a heat dissipation member (110, 300) and an operation head (20);wherein the heat dissipation member (110, 300) comprises a thermal coupling surface (111, 340);the operation head (20) comprises a cold compress member (210, 100) and a refrigeration member (200), the thermal coupling surface (111, 340) is configured to be thermally coupled with a heat generating side (220) of the refrigeration member (200) to dissipate heat generated by the refrigeration member (200); the cold compress member (210, 100) comprises a contact surface (211, 100a) and a conduction surface (212, 100b), wherein the contact surface (211, 100a) is configured to contact a skin; a cooling side (210) of the refrigeration member (220, 200) is thermally coupled with the conduction surface (212, 100b) of the cold compress member (210, 100), thereby cooling the cold compress member (210, 100); and the heat dissipation member (110, 300) extends from a head end of the skin care assembly to a trailing end of the skin care assembly;
**characterized in that** the skin care assembly further comprises a first cover (500), the first cover (500) is configured to fix the cold compress member (210, 100) and the refrigeration member (220, 200) to the heat dissipation member (110, 300), an opening (510) is defined on the first cover (500) and arranged corresponding to the cold compress member (210, 100), the heat dissipation member (300) comprises a second cover (300a) and a heat dissipation body (300b), the second cover (300a) is disposed between the heat dissipation body (300b) and the first cover (500), the second cover (300a) is fixed on the heat dissipation body (300b), and the first cover (500) is fixed on the second cover (300a).

2. The skin care assembly according to claim 1, wherein a gap is defined between an ending of the heat dissipation member (110, 300) disposed on a trailing end of the skin care assembly and an ending of the trailing end of the skin care assembly, and a size of the gap is less than one third of a length of a device body

3. The skin care assembly according to claim 1, wherein an air outlet (150) is defined on a trailing end of the skin care assembly, and a trailing end of the heat dissipation member (110) is arranged corresponding to the air outlet (150).

4. The skin care assembly according to claim 1, wherein a thermal coupling surface (111) is disposed on an end surface of a head of the heat dissipation member (110);
a recess (113) is defined on the end surface, and a bottom surface of the recess (113) is a thermal coupling surface (111);
a heat generating side (222) of the refrigeration member (220) is a heat generating surface (222), and the heat generating surface (222) is thermally coupled with the heat dissipation member (110) to dissipate heat generated by the refrigeration member (220);
the refrigeration member (220) is disposed in the recess (113), and a cooling side (221) of the refrigeration member (220) is higher than the recess (113); and
a ratio of a thickness of the refrigeration member (220) to a depth of the recess (113) is in a range from 2 to 10.

5. The skin care assembly according to claim 4, further comprising:
an installation plate (130) installed on the opening of the recess (113) and extending towards the trailing end of the skin care assembly; and
a charging port (140) disposed on an end of the installation plate (130) and exposed from the skin care assembly

6. The skin care assembly according to claim 4, further comprising: a heat pipe (120);
wherein a section of the heat pipe (120) is bent to form a bending portion (121), the bending portion (121) abuts against an end of the heat dissipation member (110) close to the operation head (20); and
the heat pipe (120) has a hollow and sealing structure and is filled with liquid, and the liquid is not full of the heat pipe (120); and
an avoidance groove (114) is defined on the heat dissipation member (110), the heat pipe (120) is arranged along an inner wall of the avoidance groove (114) and welded to the inner wall of the avoidance groove (114), and an end of the heat pipe (120) away from the bending portion (121) extends to the end of the heat dissipation member (110).

7. The skin care assembly according to claim 1, further comprising: one or more electrodes;
wherein the refrigeration member (200) is disposed between the heat dissipation member (300) and the cold compress member (100) to form a sandwich structure of the heat dissipation member (300), the refrigeration member (200), and the cold compress member (100);
a size of the opening (510) in at least one radial direction is smaller than a size of the cold compress member (100) in the radial direction, and an edge of the cold compress member (100) is pressed to the heat dissipation member (300) through an edge of the opening (510), such that the cold compress member (100) and the refrigeration member (200) are fixed on the heat dissipation member (300); and
wherein the electrodes are disposed on the edge of the opening (510) of the first cover (500), the number of the electrodes is at least two, the at least two electrodes are arranged on an outer periphery of the cold compress member (100) and spaced apart from each other

8. The skin care assembly according to claim 7, further comprising:
a first fastener (520) disposed on the first cover (500); and
a second fastener (310) disposed on the heat dissipation member (300);
wherein the second fastener (310) is snap-connected to the first fastener (520); such that the cold compress member (100) and the refrigeration member (200) are fixed on the heat dissipation member (300).

9. The skin care assembly according to claim 8, wherein the heat dissipation member comprises the thermal coupling surface (340) thermally coupled with the refrigeration member (200) and two side surfaces (320, 330) connected to two sides of the thermal coupling surface (340);
a part of the second fastener (310) is disposed a side of the thermal coupling surface (340) of the heat dissipation member (300) and snap-connected to the first fastener (520), a remaining part of the second fastener (310) extends towards the two side surfaces (320, 330) of the two sides of the thermal coupling surface (340), and the guidance portion (350, 360) is arranged on an outside of the second fastener (310); and
the first cover (500) comprises two lateral wings (550, 560) corresponding to the two side surfaces (320, 330) of the heat dissipation member (300), and the two lateral wings (550, 560) of the first cover (500) is assembled with the heat dissipation member (300) via the guidance portion (350, 360).

10. The skin care assembly according to claim 9, wherein one of the two lateral wings (550, 560) of the first cover (500) is arranged with one of a guidance groove (370) and a guidance rib (570), and the guidance groove (370) or the guidance rib (570) extends towards the heat dissipation member (300) along the inner side of the first cover (500);
one of the two side surfaces (320, 330) of the heat dissipation member (300) is arranged with the other one of the guidance groove (370) and the guidance rib (570), and the guidance groove (370) is assembled with the guidance rib (570) in a sliding manner; and
the other one of the two lateral wings (550, 560) of the first cover (500) and the other one of the two side surfaces (320, 330) of the heat dissipation member (300) are smooth.

11. The skin care assembly according to claim 1, further comprising a lead (230) connected to the refrigeration member (200);
wherein a recess (380) is defined on the second cover (300a), and the lead (230) is accommodated in the recess (380).

12. The skin care assembly according to claim 11, wherein the guidance portion (350, 360) and the second fastener (310) are disposed on the second cover (300a),a restriction hole (300c) is defined on the second cover (300a), the restriction hole (300c) is located corresponding to the opening (510) defined on the first cover (500) and is in communication with the opening (510), the refrigeration member (200) is partially disposed in the restriction hole (300c), and a distance between an edge of the refrigeration member (200) and an edge of the restriction hole (300c) is in a range from 0.5mm to lmm; and
the recess (380) is disposed along an edge of the restriction hole (300c), and a ratio of an area of the restriction hole (300c) to an area of the opening (510) is in a range from 0.8 to 1.2.

13. The skin care assembly according to claim 6, wherein the cold compress member (100) comprises a cold compress sheet (110a) and a cold-guiding sheet (120);
the cold-guiding sheet (120) is disposed between the cold compress sheet (110a) and the refrigeration member (200), the contact surface (100a) is disposed a side of the cold compress sheet (110a) away from the refrigeration member (200), the conduction surface (100b) is disposed a side of the cold-guiding sheet (120) away from the cold compress sheet (110a), a size of the cold-guiding sheet (120) in at least one radial direction is greater than a size of an opening (510) defined on the first cover (500) in the radial direction, and an edge of the cold-guiding sheet (120) is pressed to the heat dissipation member (300) by an edge of the opening (510) of the first cover (500).

14. The skin care assembly according to claim 13, wherein the cold compress sheet (110a) is disposed in the opening (510), the cold compress sheet (110a) has a first radial dimension and a second radial dimension different from the first radial dimension at different thickness positions, and the opening (510) has a third radial dimension and a fourth radial dimension different from the third radial dimension at different depth positions;
a part of the cold compress sheet (110a) having the first radial dimension is closer to the cold-guiding sheet (120) than a part of the cold compress sheet (110a) having the second radial dimension, and a part of the opening (510) having the third radial dimension is closer to the cold-guiding sheet (120) than a part of the opening (510) having the fourth radial dimension; and
the first radial dimension is smaller than the third radial dimension, the second radial dimension is smaller than the fourth radial dimension, and the second radial dimension is greater than the third radial dimension

## Patentansprüche

1. Hautpflegeanordnung, umfassend: ein Wärmeableitungselement (110, 300) und einen Arbeitskopf (20); wobei das Wärmeableitungselement (110, 300) eine thermische Kopplungsfläche (111, 340) umfasst; der Arbeitskopf (20) ein Kältekompressorelement (210, 100) und ein Kühlelement (200) umfasst, wobei die thermische Kopplungsfläche (111, 340) so konfiguriert ist, dass sie thermisch mit einer Wärmeerzeugungsseite (220) des Kühlelements (200) gekoppelt ist, um durch das Kühlelement (200) erzeugte Wärme abzuleiten; wobei das Kältekompressionselement (210, 100) eine Kontaktfläche (211, 100a) und eine Leitungsfläche (212, 100b) umfasst, wobei die Kontaktfläche (211, 100a) so konfiguriert ist, dass sie eine Haut berührt; wobei eine Kühlseite (210) des Kühlelements (220, 200) thermisch mit der Leitungsfläche (212, 100b) des Kältekompressenelements (210, 100) gekoppelt ist, wodurch das Kältekompressionselement (210, 100) gekühlt wird; und wobei sich das Wärmeableitungselement (110, 300) von einem Kopfende der Hautpflegeanordnung zu einem hinteren Ende der Hautpflegeanordnung erstreckt;
**dadurch gekennzeichnet, dass** die Hautpflegeanordnung ferner eine erste Abdeckung (500) umfasst, wobei die erste Abdeckung (500) so konfiguriert ist, dass sie das Kältekompressionselement (210, 100) und das Kühlelement (220, 200) an dem Wärmeableitungselement (110, 300) befestigt, wobei eine Öffnung (510) an der ersten Abdeckung (500) definiert und entsprechend dem Kältekompressionselement (210, 100) angeordnet ist, wobei das Wärmeableitungselement (300) eine zweite Abdeckung (300a) und einen Wärmeableitungskörper (300b) umfasst, wobei die zweite Abdeckung (300a) zwischen dem Wärmeableitungskörper (300b) und der ersten Abdeckung (500) angeordnet ist, die zweite Abdeckung (300a) an dem Wärmeableitungskörper (300b) befestigt ist und die erste Abdeckung (500) an der zweiten Abdeckung (300a) befestigt ist.

2. Hautpflegeanordnung nach Anspruch 1, wobei ein Spalt zwischen einem Ende des Wärmeableitungselements (110, 300), das an einem hinteren Ende der Hautpflegeanordnung angeordnet ist, und einem Ende des hinteren Endes der Hautpflegeanordnung definiert ist und eine Größe des Spalts weniger als ein Drittel der Länge eines Vorrichtungskörpers beträgt.

3. Hautpflegeanordnung nach Anspruch 1, wobei ein Luftauslass (150) an einem hinteren Ende der Hautpflegeanordnung definiert ist und ein hinteres Ende des Wärmeableitungselements (110) entsprechend dem Luftauslass (150) angeordnet ist.

4. Hautpflegeanordnung nach Anspruch 1, wobei eine thermische Kopplungsfläche (111) an einer Endfläche eines Kopfes des Wärmeableitungselements (110) angeordnet ist;
wobei eine Aussparung (113) an der Endfläche definiert ist und eine Bodenfläche der Aussparung (113) eine thermische Kopplungsfläche (111) ist;
wobei eine Wärmeerzeugungsseite (222) des Kühlelements (220) eine wärmeerzeugende Oberfläche (222) ist, und die wärmeerzeugende Oberfläche (222) thermisch mit dem Wärmeableitungselement (110) gekoppelt ist, um von dem Kühlelement (220) erzeugte Wärme abzuleiten;
wobei das Kühlelement (220) in der Aussparung (113) angeordnet ist, und eine Kühlseite (221) des Kühlelements (220) höher als die Aussparung (113) ist; und
wobei ein Verhältnis einer Dicke des Kühlelements (220) zu einer Tiefe der Aussparung (113) in einem Bereich von 2 bis 10 liegt.

5. Hautpflegeanordnung nach Anspruch 4, weiterhin umfassend:
eine Installationsplatte (130), die an der Öffnung der Aussparung (113) installiert ist und sich zum hinteren Ende der Hautpflegeanordnung erstreckt; und
einen Ladeanschluss (140), der an einem Ende der Installationsplatte (130) angeordnet ist und von der Hautpflegeanordnung aus zugänglich ist.

6. Hautpflegeanordnung nach Anspruch 4, ferner umfassend: ein Wärmerohr (120),
wobei ein Abschnitt des Wärmerohrs (120) gebogen ist, um einen Biegeabschnitt (121) zu bilden, wobei der Biegeabschnitt (121) an einem nahe dem Arbeitskopf (20) liegenden Ende des Wärmeableitungselements (110) anliegt; und
wobei das Wärmerohr (120) eine hohle und abdichtende Struktur aufweist und mit Flüssigkeit gefüllt ist, und die Flüssigkeit das Wärmerohr (120) nicht ausfüllt; und
wobei eine Ausweichnut (114) auf dem Wärmeableitungselement (110) definiert ist, das Wärmerohr (120) entlang einer Innenwand der Ausweichnut (114) angeordnet und mit der Innenwand der Ausweichnut (114) verschweißt ist, und wobei sich ein Ende des Wärmerohrs (120), das von dem Biegeabschnitt (121) entfernt ist, zu dem Ende des Wärmeableitungselements (110) erstreckt.

7. Hautpflegeanordnung nach Anspruch 1, weiterhin umfassend eine oder mehrere Elektroden;
wobei das Kühlelement (200) zwischen dem Wärmeableitungselement (300) und dem Kältekompressionselement (100) angeordnet ist, um eine Sandwichstruktur aus dem Wärmeableitungselement (300), dem Kühlelement (200) und dem Kältekompressionselement (100) zu bilden;
eine Größe der Öffnung (510) in mindestens einer Radialrichtung kleiner als eine Größe des Kältekompressionselements (100) in der Radialrichtung ist, und eine Kante des Kältekompressionselements (100) an das Wärmeableitungselement (300) durch einen Rand der Öffnung (510) gedrückt wird, so dass das Kältekompressionselement (100) und das Kühlelement (200) am Wärmeableitungselement (300) befestigt sind; und
wobei die Elektroden am Rand der Öffnung (510) der ersten Abdeckung (500) angeordnet sind, die Anzahl der Elektroden mindestens zwei beträgt, die mindestens zwei Elektroden an einem Außenumfang des Kältekompressionselement (100) angeordnet sind und voneinander beabstandet sind.

8. Hautpflegeanordnung nach Anspruch 7, weiterhin umfassend:
ein erstes Befestigungselement (520), das an der ersten Abdeckung (500) angeordnet ist; Und
ein zweites Befestigungselement (310), das auf dem Wärmeableitungselement (300) angeordnet ist;
wobei das zweite Befestigungselement (310) mit dem ersten Befestigungselement (520) durch Einrasten verbunden ist, so dass das Kältekompressorelement (100) und das Kühlelement (200) an dem Wärmeableitungselement (300) befestigt sind.

9. Hautpflegeanordnung nach Anspruch 8, wobei das Wärmeableitungselement die thermische Kopplungsfläche (340), die thermisch mit dem Kühlelement (200) gekoppelt ist, und zwei Seitenflächen (320, 330) umfasst, die mit zwei Seiten der thermischen Kopplungsfläche (340) verbunden sind;
ein Teil des zweiten Befestigungselements (310) auf einer Seite der thermischen Kopplungsfläche (340) des Wärmeableitungselements (300) angeordnet und mit dem ersten Befestigungselement (520) durch Einrasten verbunden ist, ein verbleibender Teil des zweiten Befestigungselements (310) sich in Richtung der beiden Seitenflächen (320, 330) der beiden Seiten der thermischen Kopplungsfläche (340) erstreckt und ein Führungsabschnitt (350, 360) an einer Außenseite des zweiten Befestigungselements (310) angeordnet ist; und
die erste Abdeckung (500) zwei Seitenflügel (550, 560) aufweist, die den beiden Seitenflächen (320, 330) des Wärmeableitungselements (300) entsprechen, und die beiden Seitenflügel (550, 560) der ersten Abdeckung (500) mit dem Wärmeableitungselement (300) über den Führungsabschnitt (350, 360) zusammengebaut sind.

10. Hautpflegeanordnung nach Anspruch 9, wobei einer der beiden Seitenflügel (550, 560) der ersten Abdeckung (500) mit einer Führungsnut (370) oder einer Führungsrippe (570) versehen ist und sich die Führungsnut (370) oder die Führungsrippe (570) entlang der Innenseite der ersten Abdeckung (500) in Richtung des Wärmeableitungselements (300) erstreckt;
eine der beiden Seitenflächen (320, 330) des Wärmeableitungselements (300) mit der anderen der Führungsnut (370) und der Führungsrippe (570) versehen ist, und die Führungsnut (370) mit der Führungsrippe (570) gleitend zusammengefügt ist; und
der andere der beiden Seitenflügel (550, 560) der ersten Abdeckung (500) und die andere der beiden Seitenflächen (320, 330) des Wärmeableitungselements (300) sind glatt.

11. Hautpflegeanordnung nach Anspruch 1, weiterhin umfassend eine Leitung (230), die mit dem Kühlelement (200) verbunden ist;
wobei an der zweiten Abdeckung (300a) eine Aussparung (380) definiert ist und die Leitung (230) in der Aussparung (380) untergebracht ist.

12. Hautpflegeanordnung nach Anspruch 11, wobei der Führungsabschnitt (350, 360) und das zweite Befestigungselement (310) an der zweiten Abdeckung (300a) angeordnet sind, wobei ein Begrenzungsloch (300c) an der zweiten Abdeckung (300a) definiert ist, wobei das Begrenzungsloch (300c) entsprechend der an der ersten Abdeckung (500) definierten Öffnung (510) angeordnet ist und mit der Öffnung (510) in Verbindung steht, wobei das Kühlelement (200) teilweise in dem Begrenzungsloch (300c) angeordnet ist und wobei ein Abstand zwischen einer Kante des Kühlelements (200) und einer Kante des Begrenzungslochs (300c) in einem Bereich von 0. 5 mm bis 1 mm liegt; und
die Aussparung (380) entlang einer Kante des Begrenzungslochs (300c) angeordnet ist und ein Verhältnis einer Fläche des Begrenzungslochs (300c) zu einer Fläche der Öffnung (510) in einem Bereich von 0,8 bis 1.2 liegt.

13. Hautpflegeanordnung nach Anspruch 6, wobei das Kältekompressenelement (100) ein Kältekompressenblech (110a) und ein Kälteleitblech (120) umfasst,
wobei das Kälteleitblech (120) zwischen dem Kältekompressionsblech (110a) und dem Kühlelement (200) angeordnet ist, die Kontaktfläche (100a) auf einer von dem Kühlelement (200) abgewandten Seite des Kältekompressionsblechs (110a) angeordnet ist, die Leitfläche (100b) auf einer von dem Kältekompressionsblech (110a) abgewandten Seite des Kälteleitblechs (120) angeordnet ist, eine Größe des Kälteleitblechs (120) in mindestens einer Radialrichtung größer ist als eine Größe einer Öffnung (510), die an der ersten Abdeckung (500) in der Radialrichtung definiert ist, und ein Rand des Kälteleitblechs (120) an das Wärmeableitungselement (300) durch einen Rand der Öffnung (510) der ersten Abdeckung (500) gedrückt wird.

14. Hautpflegeanordnung nach Anspruch 13, wobei das Kältekompressionsblech (110a) in der Öffnung (510) angeordnet ist, das Kältekompressionsblech (110a) eine erste radiale Abmessung und eine zweite radiale Abmessung, die sich von der ersten radialen Abmessung unterscheidet, an verschiedenen Dickenpositionen aufweist, und die Öffnung (510) eine dritte radiale Abmessung und eine vierte radiale Abmessung, die sich von der dritten radialen Abmessung unterscheidet, an verschiedenen Tiefenpositionen aufweist; und
ein Teil des Kältekompressionsblechs (110a) mit der ersten radialen Abmessung näher am Kälteleitblech (120) liegt als ein Teil des Kältekompressionsblechs (110a) mit der zweiten radialen Abmessung, und ein Teil der Öffnung (510) mit der dritten radialen Abmessung näher am Kälteleitblechs (120) liegt als ein Teil der Öffnung (510) mit der vierten radialen Abmessung; und
die erste radiale Abmessung kleiner als die dritte radiale Abmessung ist, die zweite radiale Abmessung kleiner als die vierte radiale Abmessung ist, und die zweite radiale Abmessung größer als die dritte radiale Abmessung ist.

## Revendications

1. Ensemble de soins de la peau, comprenant : un élément de dissipation thermique (110, 300) et une tête d'opération (20) ; dans lequel l'élément de dissipation thermique (110, 300) comprend une surface de couplage thermique (111, 340) ; la tête d'opération (20) comprend un élément de compression froide (210, 100) et un élément de réfrigération (200), la surface de couplage thermique (111, 340) est configurée pour être couplée thermiquement à un côté générateur de chaleur (220) de l'élément de réfrigération (200) pour dissiper la chaleur générée par l'élément de réfrigération (200), l'élément de compression froide (210, 100) comprend une surface de contact (211, 100a) et une surface de conduction (212, 100b), dans lequel la surface de contact (211, 100a) est configurée pour entrer en contact avec une peau, un côté de refroidissement (210) de l'élément de réfrigération (220, 200) est couplé thermiquement à la surface de conduction (212, 100b) de l'élément de compression froide (210, 100), refroidissant ainsi l'élément de compression froide (210, 100), et l'élément de dissipation thermique (110, 300) s'étend d'une extrémité de tête de l'ensemble de soins de la peau à une extrémité arrière de l'ensemble de soins de la peau ;
**caractérisé en ce que** l'ensemble de soin de la peau comprend en outre un premier couvercle (500), le premier couvercle (500) est configuré pour fixer l'élément de compression froide (210, 100) et l'élément de réfrigération (220, 200) à l'élément de dissipation thermique (110, 300), une ouverture (510) est prévue sur le premier couvercle (500) et agencée pour correspondre à l'élément de compression froide (210, 100), l'élément de dissipation thermique (300) comprend un second couvercle (300a) et un corps de dissipation de chaleur (300b), le second couvercle (300a) est disposé entre le corps de dissipation de chaleur (300b) et le premier couvercle (500), le second couvercle (300a) est fixé sur le corps de dissipation de chaleur (300b) et le premier couvercle (500) est fixé sur le second couvercle (300a).

2. Ensemble de soins de la peau selon la revendication 1, dans lequel un espace est prévu entre une extrémité de l'élément de dissipation thermique (110, 300) disposé sur une extrémité arrière de l'ensemble de soins de la peau et une queue de l'extrémité arrière de l'ensemble de soins de la peau, et une taille de l'espace est inférieure à un tiers d'une longueur d'un corps de dispositif.

3. Ensemble de soins de la peau selon la revendication 1, dans lequel une sortie d'air (150) est prévue sur une extrémité arrière de l'ensemble de soins de la peau, et une extrémité arrière de l'élément de dissipation thermique (110) est agencée pour correspondre à la sortie d'air (150).

4. Ensemble de soin de la peau selon la revendication 1, dans lequel une surface de couplage thermique (111) est disposée sur une surface d'extrémité d'une tête de l'élément de dissipation thermique (110) ;
un évidement (113) est prévu sur la surface d'extrémité, et une surface inférieure de l'évidement (113) est une surface de couplage thermique (111) ;
un côté générateur de chaleur (222) de l'élément de réfrigération (220) est une surface génératrice de chaleur (222), et la surface génératrice de chaleur (222) est couplée thermiquement à l'élément de dissipation thermique (110) pour dissiper la chaleur générée par l'élément de réfrigération (220) ;
l'élément de réfrigération (220) est disposé dans l'évidement (113), et un côté de refroidissement (221) de l'élément de réfrigération (220) est plus haut que l'évidement (113) ; et
un rapport d'une épaisseur de l'élément de réfrigération (220) à une profondeur de l'évidement (113) est compris dans une plage de 2 à 10.

5. Ensemble de soins de la peau selon la revendication 4, comprenant en outre :
une plaque d'installation (130) installée sur l'ouverture de l'évidement (113) et s'étendant vers l'extrémité arrière de l'ensemble de soins de la peau ; et
un port de charge (140) disposé sur une extrémité de la plaque d'installation (130) et exposé à partir de l'ensemble de soins de la peau.

6. Ensemble de soins de la peau selon la revendication 4, comprenant en outre : un tuyau thermique (120) ;
dans lequel une section du tuyau thermique (120) est courbée pour former une portion de coude (121), la portion de coude (121) est aboutée contre une extrémité de l'élément de dissipation thermique (110) proche de la tête d'opération (20) ; et
le tuyau thermique (120) a une structure creuse et étanche et est rempli d'un liquide, et le liquide n'est pas plein du tuyau thermique (120) ; et
une rainure d'évitement (114) est prévue sur l'élément de dissipation thermique (110), le tuyau thermique (120) est disposé le long d'une paroi intérieure de la rainure d'évitement (114) et soudé à la paroi intérieure de la rainure d'évitement (114), et une extrémité du tuyau thermique (120) éloignée de la portion de coude (121) s'étend jusqu'à l'extrémité de l'élément de dissipation thermique (110).

7. Ensemble de soin de la peau selon la revendication 1, comprenant en outre : une ou plusieurs électrodes ;
dans lequel l'élément de réfrigération (200) est disposé entre l'élément de dissipation thermique (300) et l'élément de compression froide (100) pour former une structure de sandwich pour l'élément de dissipation thermique (300), l'élément de réfrigération (200) et l'élément de compression froide (100) ;
une taille de l'ouverture (510) dans au moins une direction radiale est inférieure à une taille de l'élément de compression froide (100) dans la direction radiale, et un bord de l'élément de compression froide (100) est pressé contre l'élément de dissipation thermique (300) à travers un bord de l'ouverture (510), de sorte que l'élément de compression froide (100) et l'élément de réfrigération (200) soient fixés sur l'élément de dissipation thermique (300), et
dans lequel les électrodes sont disposées sur le bord de l'ouverture (510) du premier couvercle (500), le nombre des électrodes est d'au moins deux, les au moins deux électrodes sont disposées sur une périphérie extérieure de l'élément de compression froide (100) et espacées l'une de l'autre.

8. Ensemble de soins de la peau selon la revendication 7, comprenant en outre :
un premier fixateur (520) disposé sur le premier couvercle (500) ; et
un second fixateur (310) disposé sur l'élément de dissipation thermique (300) ;
dans lequel le second fixateur (310) est relié par enclenchement au premier fixateur (520) ; de sorte que l'élément de compression froide (100) et l'élément de réfrigération (200) soient fixés sur l'élément de dissipation thermique (300).

9. Ensemble de soin de la peau selon la revendication 8, dans lequel l'élément de dissipation thermique comprend la surface de couplage thermique (340) couplée thermiquement avec l'élément de réfrigération (200) et deux surfaces latérales (320, 330) reliées à deux côtés de la surface de couplage thermique (340) ;
une partie du second fixateur (310) est disposée sur un côté de la surface de couplage thermique (340) de l'élément de dissipation thermique (300) et reliée par enclenchement au premier fixateur (520), l'autre partie du second fixateur (310) s'étend vers les deux surfaces latérales (320, 330) des deux côtés de la surface de couplage thermique (340), et une portion de guidage (350, 360) est disposée à l'extérieur du second fixateur (310) ; et
le premier couvercle (500) comprend deux ailes latérales (550, 560) correspondant aux deux surfaces latérales (320, 330) de l'élément de dissipation thermique (300), et les deux ailes latérales (550, 560) du premier couvercle (500) sont assemblées avec l'élément de dissipation thermique (300) par l'intermédiaire de la portion de guidage (350, 360).

10. Ensemble de soin de la peau selon la revendication 9, dans lequel l'une des deux ailes latérales (550, 560) du premier couvercle (500) est disposée avec l'une d'une rainure de guidage (370) et d'une nervure de guidage (570), et la rainure de guidage (370) ou la nervure de guidage (570) s'étend vers l'élément de dissipation thermique (300) le long du côté intérieur du premier couvercle (500) ;
l'une des deux surfaces latérales (320, 330) de l'élément de dissipation thermique (300) est disposée avec l'autre de la rainure de guidage (370) et de la nervure de guidage (570), et la rainure de guidage (370) est assemblée avec la nervure de guidage (570) de manière coulissante, et
l'autre des deux ailes latérales (550, 560) du premier couvercle (500) et l'autre des deux surfaces latérales (320, 330) de l'élément de dissipation thermique (300) sont lisses.

11. Ensemble de soin de la peau selon la revendication 1, comprenant en outre un fil (230) relié à l'élément de réfrigération (200) ;
dans lequel un évidement (380) est prévu sur le second couvercle (300a), et le fil (230) est logé dans l'évidement (380).

12. Ensemble de soin de la peau selon la revendication 11, dans lequel la portion de guidage (350, 360) et le second fixateur (310) sont disposés sur le second couvercle (300a), un trou de restriction (300c) est prévu sur le second couvercle (300a),
le trou de restriction (300c) est situé pour correspondre à l'ouverture (510) prévue sur le premier couvercle (500) et est en communication avec l'ouverture (510), l'élément de réfrigération (200) est partiellement disposé dans le trou de restriction (300c), et une distance entre un bord de l'élément de réfrigération (200) et un bord du trou de restriction (300c) est compris dans une plage de 0,5 mm à 1 mm ; et
l'évidement (380) est disposé le long d'un bord du trou de restriction (300c), et un rapport d'une aire du trou de restriction (300c) à une aire de l'ouverture (510) est compris dans une plage de 0,8 à 1,2.

13. Ensemble de soins de la peau selon la revendication 6, dans lequel l'élément de compression froide (100) comprend une feuille de compression froide (110a) et une feuille de guidage froid (120) ;
la feuille de guidage froid (120) est disposée entre la feuille de compression froide (110a) et l'élément de réfrigération (200), une surface de contact (100a) est disposée sur un côté de la feuille de compression froide (110a) éloigné de l'élément de réfrigération (200), la surface de conduction (100b) est disposée sur un côté de la feuille de guidage froid (120) éloigné de la feuille de compression froide (110a), une taille de la feuille de guidage froid (120) dans au moins une direction radiale est supérieure à une taille d'une ouverture (510) prévue sur le premier couvercle (500) dans la direction radiale, et un bord de la feuille de guidage froid (120) est pressé contre l'élément de dissipation thermique (300) par un bord de l'ouverture (510) du premier couvercle (500).

14. Ensemble de soins de la peau selon la revendication 13, dans lequel la feuille de compression froide (110a) est disposée dans l'ouverture (510), la feuille de compression froide (110a) a une première dimension radiale et une deuxième dimension radiale différente de la première dimension radiale à différentes positions d'épaisseur, et l'ouverture (510) a une troisième dimension radiale et une quatrième dimension radiale différente de la troisième dimension radiale à différentes positions de profondeur ;
une partie de la feuille de compression froide (110a) ayant la première dimension radiale est plus proche de la feuille de guidage froid (120) par rapport d'une partie de la feuille de compression à froid (110a) ayant la deuxième dimension radiale, et une partie de l'ouverture (510) ayant la troisième dimension radiale est plus proche de la feuille de guidage froid (120) par rapport d'une partie de l'ouverture (510) ayant la quatrième dimension radiale ; et
la première dimension radiale est inférieure à la troisième dimension radiale, la deuxième dimension radiale est inférieure à la quatrième dimension radiale et la deuxième dimension radiale est supérieure à la troisième dimension radiale.
